# EUROPEAN PATENT APPLICATION

(11) **EP 1 223 534 A1**
(43) Date of publication of application: **17.07.2002**
(21) Application number: 02075095.6
(22) Date of filing: 11.01.2002
(51) Int. Cl.: G06F 19/00, G01N 33/53, G01N 33/68, C12P 21/06, C07K 16/18, C07K 1/12, C07K 2/00

(54) **Peptides representative of polypeptides of interest and antibodies directed thereagainst, and methods, systems and kits for generating and utilizing each**

(30) Priority: 14.01.2001 IL 14088101; 19.10.2001 US 982172
(71) Applicant: Katz, Emil Israel, Savyon 56548 (IL)
(72) Inventor: Katz, Emil Israel, Savyon 56548 (IL)
(74) Representative: Schmitz, Jean-Marie

(57) **Abstract**

A method of generating a set of amino acid sequences representative of at least one polypeptide of interest is provided. Also provided are kits and methods of using such peptides and antibodies generated thereagainst for detecting the presence absence or severity of a disease.

SEQUENCE LISTING

<110> Emil Israel Katz

<120> PEPTIDES REPRESENTATIVE OF POLYPEPTIDES OF INTEREST AND
ANTIBODIES DIRECTED THEREAGAINST, AND METHODS, SYSTEMS AND KITS FOR
GENERATING AND UTILIZING EACH

<130> 01/22283

<160> 253

<170> PatentIn version 3.1

<210> 1

<211> 7

<212> PRT

<213> Artificial sequence

<220>

<223> Computer generated synthetic peptide

<400> 1

Met Asp Leu Glu Gly Asp Arg
1               5

<210> 2

<211> 5

<212> PRT

<213> Artificial sequence

<220>

<223> Computer generated synthetic peptide

<400>  2

Asn Gly Gly Ala Lys
1               5


<210>  3

<211>  1

<212>  PRT

<213>  Artificial sequence



<220>

<223>  Computer generated synthetic peptide

<400>  3

Lys
1


<210>  4

<211>  1

<212>  PRT

<213>  Artificial sequence



<220>

<223>  Computer generated synthetic peptide

<400>  4

Lys
1


<210>  5

<211>  4

<212>  PRT

<213>  Artificial sequence



<220>

<223>  Computer generated synthetic peptide

<400>  5

Asn Phe Phe Lys
1


<210>  6

<211>  4

<212>  PRT

<213>  Artificial sequence



<220>

<223>  Computer generated synthetic peptide

<400>  6

Leu Asn Asn Lys
1


<210>  7

<211>  3

<212>  PRT

<213>  Artificial sequence



<220>

<223>  Computer generated synthetic peptide

<400>  7

Ser Glu Lys
1


<210>  8

<211>  2

<212>  PRT

<213>  Artificial sequence



<220>

<223>  Computer generated synthetic peptide

<400>  8

Asp Lys
1


<210>  9

<211>  1

<212>  PRT

<213>  Artificial sequence



<220>

<223>  Computer generated synthetic peptide

<400>  9

Lys
1


<210>  10

<211>  2

<212>  PRT

<213>  Artificial sequence



<220>

<223>  Computer generated synthetic peptide

<400>  10

Glu Lys
1


<210>  11

<211>  1

<212>  PRT

<213>  Artificial sequence



<220>

<223>  Computer generated synthetic peptide

<400>  11

Lys
1


<210>  12

<211>  10

<212>  PRT

<213>  Artificial sequence



<220>

<223>  Computer generated synthetic peptide

<400>  12

Pro Thr Val Ser Val Phe Ser Met Phe Arg
1               5                   10


<210>  13

<211>  7

<212>  PRT

<213>  Artificial sequence



<220>

<223>  Computer generated synthetic peptide

<400>  13

Tyr Ser Asn Trp Leu Asp Lys
1               5


<210>  14

<211>  47

<212>  PRT

<213>  Artificial sequence



<220>

<223>  Computer generated synthetic peptide

<400>  14

Leu Tyr Met Val Val Gly Thr Leu Ala Ala Ile Ile His Gly Ala Gly
1               5                   10                  15

Leu Pro Leu Met Met Leu Val Phe Gly Glu Met Thr Asp Ile Phe Ala
            20                  25                  30

Asn Ala Gly Asn Leu Glu Asp Leu Met Ser Asn Ile Thr Asn Arg
        35                  40                  45

<210>  15

<211>  18

<212>  PRT

<213>  Artificial sequence

<220>

<223>  Computer generated synthetic peptide

<400>  15

Ser Asp Ile Asn Asp Thr Gly Phe Phe Met Asn Leu Glu Glu Asp Met
1               5                   10                  15

Thr Arg

<210>  16

<211>  29

<212>  PRT

<213>  Artificial sequence

<220>

<223>  Computer generated synthetic peptide

<400>  16

Tyr Ala Tyr Tyr Tyr Ser Gly Ile Gly Ala Gly Val Leu Val Ala Ala
1               5                   10                  15

Tyr Ile Gln Val Ser Phe Trp Cys Leu Ala Ala Gly Arg
            20                  25

<210>  17

<211>  4

<212>  PRT

<213>  Artificial sequence

<220>

<223>  Computer generated synthetic peptide

<400>  17

Gln Ile His Lys
1

<210>  18

<211>  2

<212>  PRT

<213>  Artificial sequence

<220>

<223>  Computer generated synthetic peptide

<400>  18

Ile Arg
1

<210>  19

<211>  1

<212>  PRT

<213>  Artificial sequence

<220>

<223>  Computer generated synthetic peptide

<400>  19

Lys
1

<210>  20

<211>  8

<212>  PRT

<213>  Artificial sequence

<220>

<223>  Computer generated synthetic peptide

<400>  20

Gln Phe Phe His Ala Ile Met Arg
1               5

<210>  21

<211>  17

<212>  PRT

<213>  Artificial sequence

<220>

<223>  Computer generated synthetic peptide

<400>  21

Gln Glu Ile Gly Trp Phe Asp Val His Asp Val Gly Glu Leu Asn Thr
1               5                   10                  15

Arg

<210>  22

<211>  7

<212>  PRT

<213>  Artificial sequence

<220>

<223>  Computer generated synthetic peptide

<400>  22

Leu Thr Asp Asp Val Ser Lys
1               5

<210>  23

<211>  8

<212>  PRT

<213>  Artificial sequence

<220>

<223>  Computer generated synthetic peptide

<400>  23

Ile Asn Glu Gly Ile Gly Asp Lys
1               5

<210>  24

<211>  21

<212>  PRT

<213>  Artificial sequence

<220>

<223>  Computer generated synthetic peptide

<400>  24

Ile Gly Met Phe Phe Gln Ser Met Ala Thr Phe Phe Thr Gly Phe Ile
1               5                   10                  15

Val Gly Phe Thr Arg
            20

<210>  25

<211>  3

<212>  PRT

<213>  Artificial sequence

<220>

<223>  Computer generated synthetic peptide

<400>  25

Gly Trp Lys
1


<210>  26

<211>  21

<212>  PRT

<213>  Artificial sequence



<220>

<223>  Computer generated synthetic peptide

<400>  26

Leu Thr Leu Val Ile Leu Ala Ile Ser Pro Val Leu Gly Leu Ser Ala
1               5                   10                  15


Ala Val Trp Ala Lys
            20


<210>  27

<211>  8

<212>  PRT

<213>  Artificial sequence



<220>

<223>  Computer generated synthetic peptide

<400>  27

Ile Leu Ser Ser Phe Thr Asp Lys
1               5


<210>  28

<211>  7

<212>  PRT

<213>  Artificial sequence

<220>

<223>  Computer generated synthetic peptide

<400>  28

Glu Leu Leu Ala Tyr Ala Lys
1               5

<210>  29

<211>  13

<212>  PRT

<213>  Artificial sequence

<220>

<223>  Computer generated synthetic peptide

<400>  29

Ala Gly Ala Val Ala Glu Glu Val Leu Ala Ala Ile Arg
1               5                   10

<210>  30

<211>  9

<212>  PRT

<213>  Artificial sequence

<220>

<223>  Computer generated synthetic peptide

<400>  30

Thr Val Ile Ala Phe Gly Gly Gln Lys
1               5

<210>  31

<211>  1

<212>  PRT

<213>  Artificial sequence

<220>

<223>  Computer generated synthetic peptide

<400>  31

Lys
1

<210>  32

<211>  4

<212>  PRT

<213>  Artificial sequence

<220>

<223>  Computer generated synthetic peptide

<400>  32

Glu Leu Glu Arg
1

<210>  33

<211>  3

<212>  PRT

<213>  Artificial sequence

<220>

<223>  Computer generated synthetic peptide

<400>  33

Tyr Asn Lys
1

<210>  34

<211>  6

<212>  PRT

<213>  Artificial sequence

<220>

<223>  Computer generated synthetic peptide

<400>  34

Asn Leu Glu Glu Ala Lys
1               5

<210>  35

<211>  1

<212>  PRT

<213>  Artificial sequence

<220>

<223>  Computer generated synthetic peptide

<400>  35

Arg
1

<210>  36

<211>  4

<212>  PRT

<213>  Artificial sequence

<220>

<223>  Computer generated synthetic peptide

<400>  36

Ile Gly Ile Lys
1

<210>  37

<211>  1

<212>  PRT

<213>  Artificial sequence

<220>

<223>  Computer generated synthetic peptide

<400>  37

Lys
1

<210>  38

<211>  68

<212>  PRT

<213>  Artificial sequence

<220>

<223>  Computer generated synthetic peptide

<400>  38

Ala Ile Thr Ala Asn Ile Ser Ile Gly Ala Ala Phe Leu Leu Ile Tyr
1               5                   10                  15

Ala Ser Tyr Ala Leu Ala Phe Trp Tyr Gly Thr Thr Leu Val Leu Ser
            20                  25                  30

Gly Glu Tyr Ser Ile Gly Gln Val Leu Thr Val Phe Phe Ser Val Leu
        35                  40                  45

Ile Gly Ala Phe Ser Val Gly Gln Ala Ser Pro Ser Ile Glu Ala Phe
    50                  55                  60

Ala Asn Ala Arg
65

<210>  39

<211>  8

<212>  PRT

<213>  Artificial sequence

<220>

<223>  Computer generated synthetic peptide

<400>  39

Gly Ala Ala Tyr Glu Ile Phe Lys
1               5


<210>  40

<211>  5

<212>  PRT

<213>  Artificial sequence


<220>

<223>  Computer generated synthetic peptide

<400>  40

Ile Ile Asp Asn Lys
1               5


<210>  41

<211>  8

<212>  PRT

<213>  Artificial sequence


<220>

<223>  Computer generated synthetic peptide

<400>  41

Pro Ser Ile Asp Ser Tyr Ser Lys
1               5


<210>  42

<211>  4

<212>  PRT

<213>  Artificial sequence

<220>

<223>  Computer generated synthetic peptide

<400>  42

Ser Gly His Lys
1


<210>  43

<211>  5

<212>  PRT

<213>  Artificial sequence


<220>

<223>  Computer generated synthetic peptide

<400>  43

Pro Asp Asn Ile Lys
1               5


<210>  44

<211>  6

<212>  PRT

<213>  Artificial sequence


<220>

<223>  Computer generated synthetic peptide

<400>  44

Gly Asn Leu Glu Phe Arg
1               5


<210>  45

<211>  9

<212>  PRT

<213>  Artificial sequence

<220>

<223>  Computer generated synthetic peptide

<400>  45

Asn Val His Phe Ser Tyr Pro Ser Arg
1               5

<210>  46

<211>  1

<212>  PRT

<213>  Artificial sequence

<220>

<223>  Computer generated synthetic peptide

<400>  46

Lys
1

<210>  47

<211>  3

<212>  PRT

<213>  Artificial sequence

<220>

<223>  Computer generated synthetic peptide

<400>  47

Glu Val Lys
1

<210>  48

<211>  3

<212>  PRT

<213>  Artificial sequence

<220>

<223>  Computer generated synthetic peptide

<400>  48

Ile Leu Lys
1


<210>  49

<211>  5

<212>  PRT

<213>  Artificial sequence


<220>

<223>  Computer generated synthetic peptide

<400>  49

Gly Leu Asn Leu Lys
1               5


<210>  50

<211>  17

<212>  PRT

<213>  Artificial sequence


<220>

<223>  Computer generated synthetic peptide

<400>  50

Val Gln Ser Gly Gln Thr Val Ala Leu Val Gly Asn Ser Gly Cys Gly
1               5                   10                  15


Lys


<210>  51

<211>  9

<212>  PRT

<213>  Artificial sequence

<220>

<223>  Computer generated synthetic peptide

<400>  51

Ser Thr Thr Val Gln Leu Met Gln Arg
1               5

<210>  52

<211>  17

<212>  PRT

<213>  Artificial sequence

<220>

<223>  Computer generated synthetic peptide

<400>  52

Leu Tyr Asp Pro Thr Glu Gly Met Val Ser Val Asp Gly Gln Asp Ile
1               5                   10                  15

Arg

<210>  53

<211>  5

<212>  PRT

<213>  Artificial sequence

<220>

<223>  Computer generated synthetic peptide

<400>  53

Thr Ile Asn Val Arg
1               5

<210>  54

<211>  3

<212>  PRT

<213>  Artificial sequence

<220>

<223>  Computer generated synthetic peptide

<400>  54

Phe Leu Arg
1

<210>  55

<211>  22

<212>  PRT

<213>  Artificial sequence

<220>

<223>  Computer generated synthetic peptide

<400>  55

Glu Ile Ile Gly Val Val Ser Gln Glu Pro Val Leu Phe Ala Thr Thr
1               5                   10                  15

Ile Ala Glu Asn Ile Arg
            20

<210>  56

<211>  3

<212>  PRT

<213>  Artificial sequence

<220>

<223>  Computer generated synthetic peptide

<400>  56

Tyr Gly Arg
1

<210>  57

<211>  10

<212>  PRT

<213>  Artificial sequence

<220>

<223>  Computer generated synthetic peptide

<400>  57

Glu Asn Val Thr Met Asp Glu Ile Glu Lys
1               5                   10

<210>  58

<211>  3

<212>  PRT

<213>  Artificial sequence

<220>

<223>  Computer generated synthetic peptide

<400>  58

Ala Val Lys
1

<210>  59

<211>  10

<212>  PRT

<213>  Artificial sequence

<220>

<223>  Computer generated synthetic peptide

<400>  59

Glu Ala Asn Ala Tyr Asp Phe Ile Met Lys
1               5                   10

<210> 60

<211> 4

<212> PRT

<213> Artificial sequence

<220>

<223> Computer generated synthetic peptide

<400> 60

Leu Pro His Lys
1

<210> 61

<211> 8

<212> PRT

<213> Artificial sequence

<220>

<223> Computer generated synthetic peptide

<400> 61

Phe Asp Thr Leu Val Gly Glu Arg
1               5

<210> 62

<211> 9

<212> PRT

<213> Artificial sequence

<220>

<223> Computer generated synthetic peptide

<400> 62

Gly Ala Gln Leu Ser Gly Gly Gln Lys
1               5

<210> 63

<211> 2

<212> PRT

<213> Artificial sequence

<220>

<223> Computer generated synthetic peptide

<400> 63

Gln Arg
1

<210> 64

<211> 5

<212> PRT

<213> Artificial sequence

<220>

<223> Computer generated synthetic peptide

<400> 64

Ile Ala Ile Ala Arg
1               5

<210> 65

<211> 4

<212> PRT

<213> Artificial sequence

<220>

<223> Computer generated synthetic peptide

<400> 65

Ala Leu Val Arg
1

<210> 66

<211> 3

<212> PRT

<213> Artificial sequence

<220>

<223> Computer generated synthetic peptide

<400> 66

Asn Pro Lys
1

<210> 67

<211> 25

<212> PRT

<213> Artificial sequence

<220>

<223> Computer generated synthetic peptide

<400> 67

Ile Leu Leu Leu Asp Glu Ala Thr Ser Ala Leu Asp Thr Glu Ser Glu
1               5                   10                  15

Ala Val Val Gln Val Ala Leu Asp Lys
            20                  25

<210> 68

<211> 2

<212> PRT

<213> Artificial sequence

<220>

<223> Computer generated synthetic peptide

<400>  68

Ala Arg
1

<210>  69

<211>  1

<212>  PRT

<213>  Artificial sequence

<220>

<223>  Computer generated synthetic peptide

<400>  69

Lys
1

<210>  70

<211>  2

<212>  PRT

<213>  Artificial sequence

<220>

<223>  Computer generated synthetic peptide

<400>  70

Gly Arg
1

<210>  71

<211>  8

<212>  PRT

<213>  Artificial sequence

<220>

<223>  Computer generated synthetic peptide

<400>  71

Thr Thr Ile Val Ile Ala His Arg
1               5


<210>  72

<211>  5

<212>  PRT

<213>  Artificial sequence



<220>

<223>  Computer generated synthetic peptide

<400>  72

Leu Ser Thr Val Arg
1               5


<210>  73

<211>  16

<212>  PRT

<213>  Artificial sequence



<220>

<223>  Computer generated synthetic peptide

<400>  73

Asn Ala Asp Val Ile Ala Gly Phe Asp Asp Gly Val Ile Val Glu Lys
1               5                   10                  15


<210>  74

<211>  8

<212>  PRT

<213>  Artificial sequence



<220>

<223>  Computer generated synthetic peptide

<400>  74

Gly Asn His Asp Glu Leu Met Lys
1               5

<210>  75

<211>  2

<212>  PRT

<213>  Artificial sequence

<220>

<223>  Computer generated synthetic peptide

<400>  75

Glu Lys
1

<210>  76

<211>  5

<212>  PRT

<213>  Artificial sequence

<220>

<223>  Computer generated synthetic peptide

<400>  76

Gly Ile Tyr Phe Lys
1               5

<210>  77

<211>  21

<212>  PRT

<213>  Artificial sequence

<220>

<223>  Computer generated synthetic peptide

<400>  77

Leu Val Thr Met Gln Thr Ala Gly Asn Glu Val Glu Leu Glu Asn Ala
1               5                   10                  15

Ala Asp Glu Ser Lys
            20

<210>  78

<211>  14

<212>  PRT

<213>  Artificial sequence

<220>

<223>  Computer generated synthetic peptide

<400>  78

Ser Glu Ile Asp Ala Leu Glu Met Ser Ser Asn Asp Ser Arg
1               5                   10

<210>  79

<211>  5

<212>  PRT

<213>  Artificial sequence

<220>

<223>  Computer generated synthetic peptide

<400>  79

Ser Ser Leu Ile Arg
1               5

<210>  80

<211>  1

<212>  PRT

<213>  Artificial sequence

<220>

<223>  Computer generated synthetic peptide

<400>  80

Lys
1


<210>  81

<211>  1

<212>  PRT

<213>  Artificial sequence


<220>

<223>  Computer generated synthetic peptide

<400>  81

Arg
1


<210>  82

<211>  3

<212>  PRT

<213>  Artificial sequence


<220>

<223>  Computer generated synthetic peptide

<400>  82

Ser Thr Arg
1


<210>  83

<211>  1

<212>  PRT

<213>  Artificial sequence

<220>

<223>  Computer generated synthetic peptide

<400>  83

Arg
1


<210>  84

<211>  3

<212>  PRT

<213>  Artificial sequence


<220>

<223>  Computer generated synthetic peptide

<400>  84

Ser Val Arg
1


<210>  85

<211>  7

<212>  PRT

<213>  Artificial sequence


<220>

<223>  Computer generated synthetic peptide

<400>  85

Gly Ser Gln Ala Gln Asp Arg
1               5


<210>  86

<211>  1

<212>  PRT

<213>  Artificial sequence

<220>

<223>  Computer generated synthetic peptide

<400>  86

Lys
1


<210>  87

<211>  4

<212>  PRT

<213>  Artificial sequence


<220>

<223>  Computer generated synthetic peptide

<400>  87

Leu Ser Thr Lys
1


<210>  88

<211>  14

<212>  PRT

<213>  Artificial sequence


<220>

<223>  Computer generated synthetic peptide

<400>  88

Glu Ala Leu Asp Glu Ser Ile Pro Pro Val Ser Phe Trp Arg
1               5                   10


<210>  89

<211>  3

<212>  PRT

<213>  Artificial sequence

<220>

<223>  Computer generated synthetic peptide

<400>  89

Ile Met Lys
1


<210>  90

<211>  32

<212>  PRT

<213>  Artificial sequence



<220>

<223>  Computer generated synthetic peptide

<400>  90

Leu Asn Leu Thr Glu Trp Pro Tyr Phe Val Val Gly Val Phe Cys Ala
1               5                   10                  15


Ile Ile Asn Gly Gly Leu Gln Pro Ala Phe Ala Ile Ile Phe Ser Lys
            20                  25                  30


<210>  91

<211>  7

<212>  PRT

<213>  Artificial sequence



<220>

<223>  Computer generated synthetic peptide

<400>  91

Ile Ile Gly Val Phe Thr Arg
1               5


<210>  92

<211>  7

<212>  PRT

<213>  Artificial sequence

<220>

<223>  Computer generated synthetic peptide

<400>  92

Ile Asp Asp Pro Glu Thr Lys
1               5

<210>  93

<211>  1

<212>  PRT

<213>  Artificial sequence

<220>

<223>  Computer generated synthetic peptide

<400>  93

Arg
1

<210>  94

<211>  37

<212>  PRT

<213>  Artificial sequence

<220>

<223>  Computer generated synthetic peptide

<400>  94

Gln Asn Ser Asn Leu Phe Ser Leu Leu Phe Leu Ala Leu Gly Ile Ile
1               5                   10                  15

Ser Phe Ile Thr Phe Phe Leu Gln Gly Phe Thr Phe Gly Lys Ala Gly
            20                  25                  30

Glu Ile Leu Thr Lys
        35

<210>  95

<211>  1

<212>  PRT

<213>  Artificial sequence

<220>

<223>  Computer generated synthetic peptide

<400>  95

Arg
1

<210>  96

<211>  2

<212>  PRT

<213>  Artificial sequence

<220>

<223>  Computer generated synthetic peptide

<400>  96

Leu Arg
1

<210>  97

<211>  5

<212>  PRT

<213>  Artificial sequence

<220>

<223>  Computer generated synthetic peptide

<400>  97

Tyr Met Val Phe Arg
1               5

<210>  98

<211>  4

<212>  PRT

<213>  Artificial sequence

<220>

<223>  Computer generated synthetic peptide

<400>  98

Ser Met Leu Arg
1

<210>  99

<211>  10

<212>  PRT

<213>  Artificial sequence

<220>

<223>  Computer generated synthetic peptide

<400>  99

Gln Asp Val Ser Trp Phe Asp Asp Pro Lys
1               5                   10

<210>  100

<211>  9

<212>  PRT

<213>  Artificial sequence

<220>

<223>  Computer generated synthetic peptide

<400>  100

Asn Thr Thr Gly Ala Leu Thr Thr Arg
1               5

<210>  101

<211>  9

<212>  PRT

<213>  Artificial sequence

<220>

<223>  Computer generated synthetic peptide

<400>  101

Leu Ala Asn Asp Ala Ala Gln Val Lys
1               5

<210>  102

<211>  6

<212>  PRT

<213>  Artificial sequence

<220>

<223>  Computer generated synthetic peptide

<400>  102

Gly Ala Ile Gly Ser Arg
1               5

<210>  103

<211>  45

<212>  PRT

<213>  Artificial sequence

<220>

<223>  Computer generated synthetic peptide

<400>  103

Leu Ala Val Ile Thr Gln Asn Ile Ala Asn Leu Gly Thr Gly Ile Ile
1               5                   10                  15

Ile Ser Phe Ile Tyr Gly Trp Gln Leu Thr Leu Leu Leu Leu Ala Ile
            20                  25                  30

Val Pro Ile Ile Ala Ile Ala Gly Val Val Glu Met Lys
        35                  40                  45

<210> 104

<211> 8

<212> PRT

<213> Artificial sequence

<220>

<223> Computer generated synthetic peptide

<400> 104

Met Leu Ser Gly Gln Ala Leu Lys
1               5

<210> 105

<211> 2

<212> PRT

<213> Artificial sequence

<220>

<223> Computer generated synthetic peptide

<400> 105

Asp Lys
1

<210> 106

<211> 1

<212> PRT

<213> Artificial sequence

<220>

<223>  Computer generated synthetic peptide

<400>  106

Lys
1


<210>  107

<211>  7

<212>  PRT

<213>  Artificial sequence


<220>

<223>  Computer generated synthetic peptide

<400>  107

Glu Leu Glu Gly Ala Gly Lys
1               5


<210>  108

<211>  10

<212>  PRT

<213>  Artificial sequence


<220>

<223>  Computer generated synthetic peptide

<400>  108

Ile Ala Thr Glu Ala Ile Glu Asn Phe Arg
1               5                   10


<210>  109

<211>  10

<212>  PRT

<213>  Artificial sequence

<220>

<223>  Computer generated synthetic peptide

<400>  109

Thr Val Val Ser Leu Thr Gln Glu Gln Lys
1               5                   10


<210>  110

<211>  14

<212>  PRT

<213>  Artificial sequence


<220>

<223>  Computer generated synthetic peptide

<400>  110

Phe Glu His Met Tyr Ala Gln Ser Leu Gln Val Pro Tyr Arg
1               5                   10


<210>  111

<211>  4

<212>  PRT

<213>  Artificial sequence


<220>

<223>  Computer generated synthetic peptide

<400>  111

Asn Ser Leu Arg
1


<210>  112

<211>  1

<212>  PRT

<213>  Artificial sequence

<220>

<223>  Computer generated synthetic peptide

<400>  112

Lys
1


<210>  113

<211>  24

<212>  PRT

<213>  Artificial sequence


<220>

<223>  Computer generated synthetic peptide

<400>  113

Ala His Ile Phe Gly Ile Thr Phe Ser Phe Thr Gln Ala Met Met Tyr
1               5                   10                  15


Phe Ser Tyr Ala Gly Cys Phe Arg
            20


<210>  114

<211>  9

<212>  PRT

<213>  Artificial sequence


<220>

<223>  Computer generated synthetic peptide

<400>  114

Phe Gly Ala Tyr Leu Val Ala His Lys
1               5


<210>  115

<211>  33

<212>  PRT

<213>  Artificial sequence

<220>

<223>  Computer generated synthetic peptide

<400>  115

Leu Met Ser Phe Glu Asp Val Leu Leu Val Phe Ser Ala Val Val Phe
1               5                   10                  15

Gly Ala Met Ala Val Gly Gln Val Ser Ser Phe Ala Pro Asp Tyr Ala
            20                  25                  30

Lys

<210>  116

<211>  2

<212>  PRT

<213>  Artificial sequence

<220>

<223>  Computer generated synthetic peptide

<400>  116

Ala Lys
1

<210>  117

<211>  12

<212>  PRT

<213>  Artificial sequence

<220>

<223>  Computer generated synthetic peptide

<400>  117

Ile Ser Ala Ala His Ile Ile Met Ile Ile Glu Lys
1               5                   10

<210> 118

<211> 33

<212> PRT

<213> Artificial sequence

<220>

<223> Computer generated synthetic peptide

<400> 118

Thr Pro Leu Ile Asp Ser Tyr Ser Thr Glu Gly Leu Met Pro Asn Thr
1               5                   10                  15

Leu Glu Gly Asn Val Thr Phe Gly Glu Val Val Phe Asn Tyr Pro Thr
            20                  25                  30

Arg

<210> 119

<211> 14

<212> PRT

<213> Artificial sequence

<220>

<223> Computer generated synthetic peptide

<400> 119

Pro Asp Ile Pro Val Leu Gln Gly Leu Ser Leu Glu Val Lys
1               5                   10

<210> 120

<211> 1

<212> PRT

<213> Artificial sequence

<220>

<223>  Computer generated synthetic peptide

<400>  120

Lys
1


<210>  121

<211>  14

<212>  PRT

<213>  Artificial sequence


<220>

<223>  Computer generated synthetic peptide

<400>  121

Gly Gln Thr Leu Ala Leu Val Gly Ser Ser Gly Cys Gly Lys
1               5                   10


<210>  122

<211>  9

<212>  PRT

<213>  Artificial sequence


<220>

<223>  Computer generated synthetic peptide

<400>  122

Ser Thr Val Val Gln Leu Leu Glu Arg
1               5


<210>  123

<211>  8

<212>  PRT

<213>  Artificial sequence

<220>

<223>  Computer generated synthetic peptide

<400>  123

Phe Tyr Asp Pro Leu Ala Gly Lys
1               5


<210>  124

<211>  6

<212>  PRT

<213>  Artificial sequence


<220>

<223>  Computer generated synthetic peptide

<400>  124

Val Leu Leu Asp Gly Lys
1               5


<210>  125

<211>  3

<212>  PRT

<213>  Artificial sequence


<220>

<223>  Computer generated synthetic peptide

<400>  125

Glu Ile Lys
1


<210>  126

<211>  1

<212>  PRT

<213>  Artificial sequence

<220>

<223>  Computer generated synthetic peptide

<400>  126

Arg
1


<210>  127

<211>  7

<212>  PRT

<213>  Artificial sequence



<220>

<223>  Computer generated synthetic peptide

<400>  127

Leu Asn Val Gln Trp Leu Arg
1               5


<210>  128

<211>  28

<212>  PRT

<213>  Artificial sequence



<220>

<223>  Computer generated synthetic peptide

<400>  128

Ala His Leu Gly Ile Val Ser Gln Glu Pro Ile Leu Phe Asp Cys Ser
1               5                   10                  15


Ile Ala Glu Asn Ile Ala Tyr Gly Asp Asn Ser Arg
            20                  25


<210>  129

<211>  9

<212>  PRT

<213>  Artificial sequence

<220>

<223>  Computer generated synthetic peptide

<400>  129

Val Val Ser Gln Glu Glu Ile Val Arg
1               5

<210>  130

<211>  3

<212>  PRT

<213>  Artificial sequence

<220>

<223>  Computer generated synthetic peptide

<400>  130

Ala Ala Lys
1

<210>  131

<211>  14

<212>  PRT

<213>  Artificial sequence

<220>

<223>  Computer generated synthetic peptide

<400>  131

Glu Ala Asn Ile His Ala Phe Ile Glu Ser Leu Pro Asn Lys
1               5                   10

<210>  132

<211>  4

<212>  PRT

<213>  Artificial sequence

<220>

<223>  Computer generated synthetic peptide

<400>  132

Tyr Ser Thr Lys
1

<210>  133

<211>  4

<212>  PRT

<213>  Artificial sequence

<220>

<223>  Computer generated synthetic peptide

<400>  133

Val Gly Asp Lys
1

<210>  134

<211>  9

<212>  PRT

<213>  Artificial sequence

<220>

<223>  Computer generated synthetic peptide

<400>  134

Gly Thr Gln Leu Ser Gly Gly Gln Lys
1               5

<210>  135

<211>  2

<212>  PRT

<213>  Artificial sequence

<220>

<223>  Computer generated synthetic peptide

<400>  135

Gln Arg
1

<210>  136

<211>  5

<212>  PRT

<213>  Artificial sequence

<220>

<223>  Computer generated synthetic peptide

<400>  136

Ile Ala Ile Ala Arg
1               5

<210>  137

<211>  5

<212>  PRT

<213>  Artificial sequence

<220>

<223>  Computer generated synthetic peptide

<400>  137

Ile Ala Ile Ala Arg
1               5

<210>  138

<211>  4

<212>  PRT

<213>  Artificial sequence

<220>

<223>  Computer generated synthetic peptide

<400>  138

Ala Leu Val Arg
1

<210>  139

<211>  20

<212>  PRT

<213>  Artificial sequence

<220>

<223>  Computer generated synthetic peptide

<400>  139

Gln Pro His Ile Leu Leu Leu Asp Glu Ala Thr Ser Ala Leu Asp Thr
1               5                   10                  15

Glu Ser Glu Lys
            20

<210>  140

<211>  8

<212>  PRT

<213>  Artificial sequence

<220>

<223>  Computer generated synthetic peptide

<400>  140

Val Val Gln Glu Ala Leu Asp Lys
1               5

<210>  141

<211>  2

<212>  PRT

<213>  Artificial sequence

<220>

<223>  Computer generated synthetic peptide

<400>  141

Ala Arg
1

<210>  142

<211>  3

<212>  PRT

<213>  Artificial sequence

<220>

<223>  Computer generated synthetic peptide

<400>  142

Glu Gly Arg
1

<210>  143

<211>  8

<212>  PRT

<213>  Artificial sequence

<220>

<223>  Computer generated synthetic peptide

<400>  143

Thr Cys Ile Val Ile Ala His Arg
1               5

<210>  144

<211>  17

<212>  PRT

<213>  Artificial sequence

<220>

<223>  Computer generated synthetic peptide

<400>  144

Leu Ser Thr Ile Gln Asn Ala Asp Leu Ile Val Val Phe Gln Asn Gly
1               5                   10                  15

Arg

<210>  145

<211>  2

<212>  PRT

<213>  Artificial sequence

<220>

<223>  Computer generated synthetic peptide

<400>  145

Val Lys
1

<210>  146

<211>  12

<212>  PRT

<213>  Artificial sequence

<220>

<223>  Computer generated synthetic peptide

<400>  146

Glu His Gly Thr His Gln Gln Leu Leu Ala Gln Lys
1               5                   10

<210> 147

<211> 14

<212> PRT

<213> Artificial sequence

<220>

<223> Computer generated synthetic peptide

<400> 147

Gly Ile Tyr Phe Ser Met Val Ser Val Gln Ala Gly Thr Lys
1               5                   10

<210> 148

<211> 1

<212> PRT

<213> Artificial sequence

<220>

<223> Computer generated synthetic peptide

<400> 148

Arg
1

<210> 149

<211> 1

<212> PRT

<213> Artificial sequence

<220>

<223> Computer generated synthetic peptide

<400> 149

Gln
1

<210>  150

<211>  29

<212>  PRT

<213>  Artificial sequence

<220>

<223>  Computer generated synthetic peptide

<400>  150

Met Ser Ser Ser Asn Val Glu Val Phe Ile Pro Val Ser Gln Gly Asn
1               5                   10                  15

Thr Asn Gly Phe Pro Ala Thr Val Ser Asn Asp Leu Lys
            20                  25

<210>  151

<211>  16

<212>  PRT

<213>  Artificial sequence

<220>

<223>  Computer generated synthetic peptide

<400>  151

Ala Phe Thr Glu Gly Ala Val Leu Ser Phe His Asn Ile Cys Tyr Arg
1               5                   10                  15

<210>  152

<211>  2

<212>  PRT

<213>  Artificial sequence

<220>

<223>  Computer generated synthetic peptide

<400>  152

Val Lys
1

<210>  153

<211>  2

<212>  PRT

<213>  Artificial sequence

<220>

<223>  Computer generated synthetic peptide

<400>  153

Leu Lys
1

<210>  154

<211>  7

<212>  PRT

<213>  Artificial sequence

<220>

<223>  Computer generated synthetic peptide

<400>  154

Ser Gly Phe Leu Pro Cys Arg
1               5

<210>  155

<211>  1

<212>  PRT

<213>  Artificial sequence

<220>

<223>  Computer generated synthetic peptide

<400>  155

Lys
1


<210>  156

<211>  4

<212>  PRT

<213>  Artificial sequence


<220>

<223>  Computer generated synthetic peptide

<400>  156

Pro Val Glu Lys
1


<210>  157

<211>  11

<212>  PRT

<213>  Artificial sequence


<220>

<223>  Computer generated synthetic peptide

<400>  157

Glu Ile Leu Ser Asn Ile Asn Gly Ile Met Lys
1               5                   10


<210>  158

<211>  14

<212>  PRT

<213>  Artificial sequence


<220>

<223>  Computer generated synthetic peptide

<400>  158

Pro Gly Leu Asn Ala Ile Leu Gly Pro Thr Gly Gly Gly Lys
1               5                   10

<210>  159

<211>  10

<212>  PRT

<213>  Artificial sequence

<220>

<223>  Computer generated synthetic peptide

<400>  159

Ser Ser Leu Leu Asp Val Leu Ala Ala Arg
1               5                   10

<210>  160

<211>  1

<212>  PRT

<213>  Artificial sequence

<220>

<223>  Computer generated synthetic peptide

<400>  160

Lys
1

<210>  161

<211>  16

<212>  PRT

<213>  Artificial sequence

<220>

<223>  Computer generated synthetic peptide

<400> 161

Asp Pro Ser Gly Leu Ser Gly Asp Val Leu Ile Asn Gly Ala Pro Arg
1               5                   10                  15

<210> 162

<211> 5

<212> PRT

<213> Artificial sequence

<220>

<223> Computer generated synthetic peptide

<400> 162

Pro Ala Asn Phe Lys
1               5

<210> 163

<211> 19

<212> PRT

<213> Artificial sequence

<220>

<223> Computer generated synthetic peptide

<400> 163

Cys Asn Ser Gly Tyr Val Val Gln Asp Asp Val Val Met Gly Thr Leu
1               5                   10                  15

Thr Val Arg

<210> 164

<211> 10

<212> PRT

<213> Artificial sequence

<220>

<223>  Computer generated synthetic peptide

<400>  164

Glu Asn Leu Gln Phe Ser Ala Ala Leu Arg
1               5                   10


<210>  165

<211>  10

<212>  PRT

<213>  Artificial sequence


<220>

<223>  Computer generated synthetic peptide

<400>  165

Leu Ala Thr Thr Met Thr Asn His Glu Lys
1               5                   10


<210>  166

<211>  3

<212>  PRT

<213>  Artificial sequence


<220>

<223>  Computer generated synthetic peptide

<400>  166

Asn Glu Arg
1


<210>  167

<211>  3

<212>  PRT

<213>  Artificial sequence

<220>

<223>  Computer generated synthetic peptide

<400>  167

Ile Asn Arg
1


<210>  168

<211>  9

<212>  PRT

<213>  Artificial sequence


<220>

<223>  Computer generated synthetic peptide

<400>  168

Val Ile Glu Glu Leu Gly Leu Asp Lys
1               5


<210>  169

<211>  5

<212>  PRT

<213>  Artificial sequence


<220>

<223>  Computer generated synthetic peptide

<400>  169

Val Ala Asp Ser Lys
1               5


<210>  170

<211>  7

<212>  PRT

<213>  Artificial sequence

<220>

<223>  Computer generated synthetic peptide

<400>  170

Val Gly Thr Gln Phe Ile Arg
1               5

<210>  171

<211>  7

<212>  PRT

<213>  Artificial sequence

<220>

<223>  Computer generated synthetic peptide

<400>  171

Gly Val Ser Gly Gly Glu Arg
1               5

<210>  172

<211>  1

<212>  PRT

<213>  Artificial sequence

<220>

<223>  Computer generated synthetic peptide

<400>  172

Lys
1

<210>  173

<211>  1

<212>  PRT

<213>  Artificial sequence

<220>

<223>  Computer generated synthetic peptide

<400>  173

Arg
1


<210>  174

<211>  36

<212>  PRT

<213>  Artificial sequence



<220>

<223>  Computer generated synthetic peptide

<400>  174

Thr Ser Ile Gly Met Glu Leu Ile Thr Asp Pro Ser Ile Leu Ser Leu
1               5                   10                  15


Asp Glu Pro Thr Thr Gly Leu Asp Ser Ser Thr Ala Asn Ala Val Leu
            20                  25                  30


Leu Leu Leu Lys
        35


<210>  175

<211>  1

<212>  PRT

<213>  Artificial sequence



<220>

<223>  Computer generated synthetic peptide

<400>  175

Arg
1


<210>  176

<211>  3

<212>  PRT

<213>  Artificial sequence



<220>

<223>  Computer generated synthetic peptide

<400>  176

Met Ser Lys
1


<210>  177

<211>  3

<212>  PRT

<213>  Artificial sequence



<220>

<223>  Computer generated synthetic peptide

<400>  177

Gln Gly Arg
1


<210>  178

<211>  10

<212>  PRT

<213>  Artificial sequence



<220>

<223>  Computer generated synthetic peptide

<400>  178

Thr Ile Ile Phe Ser Ile His Gln Pro Arg
1               5                   10


<210>  179

<211>  5

<212>  PRT

<213>  Artificial sequence

<220>

<223>  Computer generated synthetic peptide

<400>  179

Tyr Ser Ile Phe Lys
1               5

<210>  180

<211>  12

<212>  PRT

<213>  Artificial sequence

<220>

<223>  Computer generated synthetic peptide

<400>  180

Leu Phe Asp Ser Leu Thr Leu Leu Ala Ser Gly Arg
1               5                   10

<210>  181

<211>  46

<212>  PRT

<213>  Artificial sequence

<220>

<223>  Computer generated synthetic peptide

<400>  181

Leu Met Phe His Gly Pro Ala Gln Glu Ala Leu Gly Tyr Phe Glu Ser
1               5                   10                  15

Ala Gly Tyr His Cys Glu Ala Tyr Asn Asn Pro Ala Asp Phe Phe Leu
            20                  25                  30

Asp Ile Ile Asn Gly Asp Ser Thr Ala Val Ala Leu Asn Arg
        35                  40                  45

<210>  182

<211>  5

<212>  PRT

<213>  Artificial sequence

<220>

<223>  Computer generated synthetic peptide

<400>  182

Glu Glu Asp Phe Lys
1               5

<210>  183

<211>  9

<212>  PRT

<213>  Artificial sequence

<220>

<223>  Computer generated synthetic peptide

<400>  183

Ala Thr Glu Ile Ile Glu Pro Ser Lys
1               5

<210>  184

<211>  3

<212>  PRT

<213>  Artificial sequence

<220>

<223>  Computer generated synthetic peptide

<400>  184

Gln Asp Lys
1


<210>  185

<211>  5

<212>  PRT

<213>  Artificial sequence



<220>

<223>  Computer generated synthetic peptide

<400>  185

Pro Leu Ile Glu Lys
1               5


<210>  186

<211>  12

<212>  PRT

<213>  Artificial sequence



<220>

<223>  Computer generated synthetic peptide

<400>  186

Leu Ala Glu Ile Tyr Val Asn Ser Ser Phe Tyr Lys
1               5                   10


<210>  187

<211>  3

<212>  PRT

<213>  Artificial sequence



<220>

<223>  Computer generated synthetic peptide

<400> 187

Glu Thr Lys
1

<210> 188

<211> 11

<212> PRT

<213> Artificial sequence

<220>

<223> Computer generated synthetic peptide

<400> 188

Ala Glu Leu His Gln Leu Ser Gly Gly Glu Lys
1               5                   10

<210> 189

<211> 1

<212> PRT

<213> Artificial sequence

<220>

<223> Computer generated synthetic peptide

<400> 189

Lys
1

<210> 190

<211> 1

<212> PRT

<213> Artificial sequence

<220>

<223> Computer generated synthetic peptide

<400>  190

Lys
1


<210>  191

<211>  1

<212>  PRT

<213>  Artificial sequence


<220>

<223>  Computer generated synthetic peptide

<400>  191

Lys
1


<210>  192

<211>  5

<212>  PRT

<213>  Artificial sequence


<220>

<223>  Computer generated synthetic peptide

<400>  192

Ile Thr Val Phe Lys
1               5


<210>  193

<211>  13

<212>  PRT

<213>  Artificial sequence


<220>

<223>  Computer generated synthetic peptide

<400> 193

Glu Ile Ser Tyr Thr Thr Ser Phe Cys His Gln Leu Arg
1               5                   10

<210> 194

<211> 4

<212> PRT

<213> Artificial sequence

<220>

<223> Computer generated synthetic peptide

<400> 194

Trp Val Ser Lys
1

<210> 195

<211> 1

<212> PRT

<213> Artificial sequence

<220>

<223> Computer generated synthetic peptide

<400> 195

Arg
1

<210> 196

<211> 3

<212> PRT

<213> Artificial sequence

<220>

<223> Computer generated synthetic peptide

<400>  196

Ser Phe Lys
1


<210>  197

<211>  31

<212>  PRT

<213>  Artificial sequence



<220>

<223>  Computer generated synthetic peptide

<400>  197

Asn Leu Leu Gly Asn Pro Gln Ala Ser Ile Ala Gln Ile Ile Val Thr
1               5                   10                  15

Val Val Leu Gly Leu Val Ile Gly Ala Ile Tyr Phe Gly Leu Lys
            20                  25                  30


<210>  198

<211>  9

<212>  PRT

<213>  Artificial sequence



<220>

<223>  Computer generated synthetic peptide

<400>  198

Asn Asp Ser Thr Gly Ile Gln Asn Arg
1               5


<210>  199

<211>  26

<212>  PRT

<213>  Artificial sequence

<220>

<223>  Computer generated synthetic peptide

<400>  199

Ala Gly Val Leu Phe Phe Leu Thr Thr Asn Gln Cys Phe Ser Ser Val
1               5                   10                  15

Ser Ala Val Glu Leu Phe Val Val Glu Lys
            20                  25

<210>  200

<211>  1

<212>  PRT

<213>  Artificial sequence

<220>

<223>  Computer generated synthetic peptide

<400>  200

Lys
1

<210>  201

<211>  12

<212>  PRT

<213>  Artificial sequence

<220>

<223>  Computer generated synthetic peptide

<400>  201

Leu Phe Ile His Glu Tyr Ile Ser Gly Tyr Tyr Arg
1               5                   10

<210>  202

<211>  8

<212>  PRT

<213>  Artificial sequence

<220>

<223>  Computer generated synthetic peptide

<400>  202

Val Ser Ser Tyr Phe Leu Gly Lys
1               5

<210>  203

<211>  9

<212>  PRT

<213>  Artificial sequence

<220>

<223>  Computer generated synthetic peptide

<400>  203

Leu Leu Ser Asp Leu Leu Pro Met Arg
1               5

<210>  204

<211>  18

<212>  PRT

<213>  Artificial sequence

<220>

<223>  Computer generated synthetic peptide

<400>  204

Met Leu Pro Ser Ile Ile Phe Thr Cys Ile Val Tyr Phe Met Leu Gly
1               5                   10                  15

Leu Lys

<210>  205

<211>  2

<212>  PRT

<213>  Artificial sequence

<220>

<223>  Computer generated synthetic peptide

<400>  205

Pro Lys
1

<210>  206

<211>  73

<212>  PRT

<213>  Artificial sequence

<220>

<223>  Computer generated synthetic peptide

<400>  206

Ala Asp Ala Phe Phe Val Met Met Phe Thr Leu Met Met Val Ala Tyr
1               5                   10                  15

Ser Ala Ser Ser Met Ala Leu Ala Ile Ala Ala Gly Gln Ser Val Val
            20                  25                  30

Ser Val Ala Thr Leu Leu Met Thr Ile Cys Phe Val Phe Met Met Ile
        35                  40                  45

Phe Ser Gly Leu Leu Val Asn Leu Thr Thr Ile Ala Ser Trp Leu Ser
    50                  55                  60

Trp Leu Gln Tyr Phe Ser Ile Pro Arg
65                  70

<210>  207

<211>  41

<212>  PRT

<213>  Artificial sequence

<220>

<223>  Computer generated synthetic peptide

<400>  207

Tyr Gly Phe Thr Ala Leu Gln His Asn Glu Phe Leu Gly Gln Asn Phe
1               5                   10                  15

Cys Pro Gly Leu Asn Ala Thr Gly Asn Asn Pro Cys Asn Tyr Ala Thr
            20                  25                  30

Cys Thr Gly Glu Glu Tyr Leu Val Lys
        35                  40

<210>  208

<211>  12

<212>  PRT

<213>  Artificial sequence

<220>

<223>  Computer generated synthetic peptide

<400>  208

Gln Gly Ile Asp Leu Ser Pro Trp Gly Leu Trp Lys
1               5                   10

<210>  209

<211>  19

<212>  PRT

<213>  Artificial sequence

<220>

<223>  Computer generated synthetic peptide

<400>  209

Asn His Val Ala Leu Ala Cys Met Ile Val Ile Phe Leu Thr Ile Ala
1               5                   10                  15

Tyr Leu Lys

<210> 210

<211> 5

<212> PRT

<213> Artificial sequence

<220>

<223> Computer generated synthetic peptide

<400> 210

Leu Leu Phe Leu Lys
1               5

<210> 211

<211> 1

<212> PRT

<213> Artificial sequence

<220>

<223> Computer generated synthetic peptide

<400> 211

Lys
1

<210> 212

<211> 2

<212> PRT

<213> Artificial sequence

<220>

<223> Computer generated synthetic peptide

<400>  212

Tyr Ser
1

<210>  213

<211>  47

<212>  PRT

<213>  Artificial sequence

<220>

<223>  Computer generated synthetic peptide

<400>  213

Leu Tyr Met Val Val Gly Thr Leu Ala Ala Ile Ile His Gly Ala Gly
1               5                   10                  15

Leu Pro Leu Met Met Leu Val Phe Gly Glu Met Thr Asp Ile Phe Ala
            20                  25                  30

Asn Ala Gly Asn Leu Glu Asp Leu Met Ser Asn Ile Thr Asn Arg
        35                  40                  45

<210>  214

<211>  18

<212>  PRT

<213>  Artificial sequence

<220>

<223>  Computer generated synthetic peptide

<400>  214

Ser Asp Ile Asn Asp Thr Gly Phe Phe Met Asn Leu Glu Glu Asp Met
1               5                   10                  15

Thr Arg

<210>  215

<211>  29

<212>  PRT

<213>  Artificial sequence

<220>

<223>  Computer generated synthetic peptide

<400>  215

Tyr Ala Tyr Tyr Tyr Ser Gly Ile Gly Ala Gly Val Leu Val Ala Ala
1               5                   10                  15

Tyr Ile Gln Val Ser Phe Trp Cys Leu Ala Ala Gly Arg
            20                  25

<210>  216

<211>  21

<212>  PRT

<213>  Artificial sequence

<220>

<223>  Computer generated synthetic peptide

<400>  216

Ile Gly Met Phe Phe Gln Ser Met Ala Thr Phe Phe Thr Gly Phe Ile
1               5                   10                  15

Val Gly Phe Thr Arg
            20

<210>  217

<211>  21

<212>  PRT

<213>  Artificial sequence

<220>

<223>  Computer generated synthetic peptide

<400>  217

Leu Thr Leu Val Ile Leu Ala Ile Ser Pro Val Leu Gly Leu Ser Ala
1               5                   10                  15

Ala Val Trp Ala Lys
            20

<210>  218

<211>  68

<212>  PRT

<213>  Artificial sequence

<220>

<223>  Computer generated synthetic peptide

<400>  218

Ala Ile Thr Ala Asn Ile Ser Ile Gly Ala Ala Phe Leu Leu Ile Tyr
1               5                   10                  15

Ala Ser Tyr Ala Leu Ala Phe Trp Tyr Gly Thr Thr Leu Val Leu Ser
            20                  25                  30

Gly Glu Tyr Ser Ile Gly Gln Val Leu Thr Val Phe Phe Ser Val Leu
        35                  40                  45

Ile Gly Ala Phe Ser Val Gly Gln Ala Ser Pro Ser Ile Glu Ala Phe
    50                  55                  60

Ala Asn Ala Arg
65

<210>  219

<211>  17

<212>  PRT

<213>  Artificial sequence

<220>

<223>  Computer generated synthetic peptide

<400>  219

Leu Tyr Asp Pro Thr Glu Gly Met Val Ser Val Asp Gly Gln Asp Ile
1               5                   10                  15

Arg

<210>  220

<211>  25

<212>  PRT

<213>  Artificial sequence

<220>

<223>  Computer generated synthetic peptide

<400>  220

Ile Leu Leu Leu Asp Glu Ala Thr Ser Ala Leu Asp Thr Glu Ser Glu
1               5                   10                  15

Ala Val Val Gln Val Ala Leu Asp Lys
            20                  25

<210>  221

<211>  16

<212>  PRT

<213>  Artificial sequence

<220>

<223>  Computer generated synthetic peptide

<400>  221

Asn Ala Asp Val Ile Ala Gly Phe Asp Asp Gly Val Ile Val Glu Lys
1               5                   10                  15

<210>  222

<211>  21

<212>  PRT

<213>  Artificial sequence

<220>

<223>  Computer generated synthetic peptide

<400>  222

Leu Val Thr Met Gln Thr Ala Gly Asn Glu Val Glu Leu Glu Asn Ala
1               5                   10                  15

Ala Asp Glu Ser Lys
            20

<210>  223

<211>  14

<212>  PRT

<213>  Artificial sequence

<220>

<223>  Computer generated synthetic peptide

<400>  223

Ser Glu Ile Asp Ala Leu Glu Met Ser Ser Asn Asp Ser Arg
1               5                   10

<210>  224

<211>  14

<212>  PRT

<213>  Artificial sequence

<220>

<223>  Computer generated synthetic peptide

<400>  224

Glu Ala Leu Asp Glu Ser Ile Pro Pro Val Ser Phe Trp Arg
1               5                   10

<210>  225

<211>  32

<212>  PRT

<213>  Artificial sequence



<220>

<223>  Computer generated synthetic peptide

<400>  225

Leu Asn Leu Thr Glu Trp Pro Tyr Phe Val Val Gly Val Phe Cys Ala
1               5                   10                  15


Ile Ile Asn Gly Gly Leu Gln Pro Ala Phe Ala Ile Ile Phe Ser Lys
            20                  25                  30


<210>  226

<211>  28

<212>  PRT

<213>  Artificial sequence



<220>

<223>  Computer generated synthetic peptide

<400>  226

Gln Asn Ser Asn Leu Phe Ser Leu Leu Phe Leu Ala Leu Gly Ile Ile
1               5                   10                  15


Ser Phe Ile Thr Phe Phe Leu Gln Gly Phe Thr Lys
            20                  25


<210>  227

<211>  45

<212>  PRT

<213>  Artificial sequence



<220>

<223>  Computer generated synthetic peptide

<400>  227

Leu Ala Val Ile Thr Gln Asn Ile Ala Asn Leu Gly Thr Gly Ile Ile
1               5                   10                  15

Ile Ser Phe Ile Tyr Gly Trp Gln Leu Thr Leu Leu Leu Leu Ala Ile
            20                  25                  30

Val Pro Ile Ile Ala Ile Ala Gly Val Val Glu Met Lys
        35                  40                  45

<210>  228

<211>  14

<212>  PRT

<213>  Artificial sequence

<220>

<223>  Computer generated synthetic peptide

<400>  228

Phe Glu His Met Tyr Ala Gln Ser Leu Gln Val Pro Tyr Arg
1               5                   10

<210>  229

<211>  24

<212>  PRT

<213>  Artificial sequence

<220>

<223>  Computer generated synthetic peptide

<400>  229

Ala His Ile Phe Gly Ile Thr Phe Ser Phe Thr Gln Ala Met Met Tyr
1               5                   10                  15

Phe Ser Tyr Ala Gly Cys Phe Arg
            20

<210>  230

<211>  32

<212>  PRT

<213>  Artificial sequence



<220>

<223>  Computer generated synthetic peptide

<400>  230

Leu Met Ser Phe Glu Asp Val Leu Leu Val Phe Ser Ala Val Val Phe
1               5                   10                  15

Gly Ala Met Ala Val Gly Gln Ser Ser Phe Ala Pro Asp Tyr Ala Lys
            20                  25                  30

<210>  231

<211>  33

<212>  PRT

<213>  Artificial sequence



<220>

<223>  Computer generated synthetic peptide

<400>  231

Thr Pro Leu Ile Asp Ser Tyr Ser Thr Glu Gly Leu Met Pro Asn Thr
1               5                   10                  15

Leu Glu Gly Asn Val Thr Phe Gly Glu Val Val Phe Asn Tyr Pro Thr
            20                  25                  30

Arg


<210>  232

<211>  14

<212>  PRT

<213>  Artificial sequence

<220>

<223>  Computer generated synthetic peptide

<400>  232

Glu Ala Asn Ile His Ala Phe Ile Glu Ser Leu Pro Asn Lys
1               5                   10

<210>  233

<211>  14

<212>  PRT

<213>  Artificial sequence

<220>

<223>  Computer generated synthetic peptide

<400>  233

Gly Ile Tyr Phe Ser Met Val Ser Val Gln Ala Gly Thr Lys
1               5                   10

<210>  234

<211>  29

<212>  PRT

<213>  Artificial sequence

<220>

<223>  Computer generated synthetic peptide

<400>  234

Met Ser Ser Ser Asn Val Glu Val Phe Ile Pro Val Ser Gln Gly Asn
1               5                   10                  15

Thr Asn Gly Phe Pro Ala Thr Val Ser Asn Asp Leu Lys
            20                  25

<210>  235

<211>  16

<212>  PRT

<213>  Artificial sequence

<220>

<223>  Computer generated synthetic peptide

<400>  235

Ala Phe Thr Glu Gly Ala Val Leu Ser Phe His Asn Ile Cys Tyr Arg
1               5                   10                  15

<210>  236

<211>  25

<212>  PRT

<213>  Artificial sequence

<220>

<223>  Computer generated synthetic peptide

<400>  236

Glu Ile Leu Ser Asn Ile Asn Gly Ile Met Lys Pro Gly Leu Asn Ala
1               5                   10                  15

Ile Leu Gly Pro Thr Gly Gly Gly Lys
            20                  25

<210>  237

<211>  21

<212>  PRT

<213>  Artificial sequence

<220>

<223>  Computer generated synthetic peptide

<400>  237

Asp Pro Ser Gly Leu Ser Gly Asp Val Leu Ile Asn Gly Ala Pro Arg
1               5                   10                  15

Pro Ala Asn Phe Lys
            20

<210> 238

<211> 19

<212> PRT

<213> Artificial sequence

<220>

<223> Computer generated synthetic peptide

<400> 238

Cys Asn Ser Gly Tyr Val Val Gln Asp Asp Val Val Met Gly Thr Leu
1               5                   10                  15

Thr Val Arg

<210> 239

<211> 10

<212> PRT

<213> Artificial sequence

<220>

<223> Computer generated synthetic peptide

<400> 239

Glu Asn Leu Gln Phe Ser Ala Ala Leu Arg
1               5                   10

<210> 240

<211> 10

<212> PRT

<213> Artificial sequence

<220>

<223> Computer generated synthetic peptide

<400>  240

Leu Ala Thr Thr Met Thr Asn His Glu Lys
1               5                   10

<210>  241

<211>  36

<212>  PRT

<213>  Artificial sequence

<220>

<223>  Computer generated synthetic peptide

<400>  241

Thr Ser Ile Gly Met Glu Leu Ile Thr Asp Pro Ser Ile Leu Ser Leu
1               5                   10                  15

Asp Glu Pro Thr Thr Gly Leu Asp Ser Ser Thr Ala Asn Ala Val Leu
            20                  25                  30

Leu Leu Leu Lys
        35

<210>  242

<211>  10

<212>  PRT

<213>  Artificial sequence

<220>

<223>  Computer generated synthetic peptide

<400>  242

Thr Ile Ile Phe Ser Ile His Gln Pro Arg
1               5                   10

<210>  243

<211>  12

<212>  PRT

<213>  Artificial sequence

<220>

<223>  Computer generated synthetic peptide

<400>  243

Leu Phe Asp Ser Leu Thr Leu Leu Ala Ser Gly Arg
1               5                   10

<210>  244

<211>  46

<212>  PRT

<213>  Artificial sequence

<220>

<223>  Computer generated synthetic peptide

<400>  244

Leu Met Phe His Gly Pro Ala Gln Glu Ala Leu Gly Tyr Phe Glu Ser
1               5                   10                  15

Ala Gly Tyr His Cys Glu Ala Tyr Asn Asn Pro Ala Asp Phe Phe Leu
            20                  25                  30

Asp Ile Ile Asn Gly Asp Ser Thr Ala Val Ala Leu Asn Arg
        35                  40                  45

<210>  245

<211>  12

<212>  PRT

<213>  Artificial sequence

<220>

<223>  Computer generated synthetic peptide

<400>  245

Leu Ala Glu Ile Tyr Val Asn Ser Ser Phe Tyr Lys
1               5                   10

<210> 246

<211> 13

<212> PRT

<213> Artificial sequence

<220>

<223> Computer generated synthetic peptide

<400> 246

Glu Ile Ser Tyr Thr Thr Ser Phe Cys His Gln Leu Arg
1               5                   10

<210> 247

<211> 31

<212> PRT

<213> Artificial sequence

<220>

<223> Computer generated synthetic peptide

<400> 247

Asn Leu Leu Gly Asn Pro Gln Ala Ser Ile Ala Gln Ile Ile Val Thr
1               5                   10                  15

Val Val Leu Gly Leu Val Ile Gly Ala Ile Tyr Phe Gly Leu Lys
            20                  25                  30

<210> 248

<211> 26

<212> PRT

<213> Artificial sequence

<220>

<223> Computer generated synthetic peptide

<400>  248

Ala Gly Val Leu Phe Phe Leu Thr Thr Asn Gln Cys Phe Ser Ser Val
1               5                   10                  15

Ser Ala Val Glu Leu Phe Val Val Glu Lys
            20                  25

<210>  249

<211>  12

<212>  PRT

<213>  Artificial sequence

<220>

<223>  Computer generated synthetic peptide

<400>  249

Leu Phe Ile His Glu Tyr Ile Ser Gly Tyr Tyr Arg
1               5                   10

<210>  250

<211>  72

<212>  PRT

<213>  Artificial sequence

<220>

<223>  Computer generated synthetic peptide

<400>  250

Ala Asp Ala Phe Phe Val Met Met Phe Thr Leu Met Met Val Ala Tyr
1               5                   10                  15

Ser Ala Ser Ser Met Ala Leu Ala Ile Ala Ala Gly Gln Ser Val Ser
            20                  25                  30

Val Ala Thr Leu Leu Met Thr Ile Cys Phe Val Phe Met Met Ile Phe
        35                  40                  45

Ser Gly Leu Leu Val Asn Leu Thr Thr Ile Ala Ser Trp Leu Ser Trp
    50                  55                  60

Leu Gln Tyr Phe Ser Ile Pro Arg
65                  70

<210>  251

<211>  41

<212>  PRT

<213>  Artificial sequence

<220>

<223>  Computer generated synthetic peptide

<400>  251

Tyr Gly Phe Thr Ala Leu Gln His Asn Glu Phe Leu Gly Gln Asn Phe
1               5                   10                  15

Cys Pro Gly Leu Asn Ala Thr Gly Asn Asn Pro Cys Asn Tyr Ala Thr
            20                  25                  30

Cys Thr Gly Glu Glu Tyr Leu Val Lys
        35                  40

<210>  252

<211>  12

<212>  PRT

<213>  Artificial sequence

<220>

<223>  Computer generated synthetic peptide

<400>  252

Gln Gly Ile Asp Leu Ser Pro Trp Gly Leu Trp Lys
1               5                   10

<210>  253

<211>  19

<212>  PRT

<213>  Artificial sequence

<220>

<223>  Computer generated synthetic peptide

<400>  253

Asn His Val Ala Leu Ala Cys Met Ile Val Ile Phe Leu Thr Ile Ala
1               5                   10                  15

Tyr Leu Lys

## Description

### FIELD AND BACKGROUND OF THE INVENTION

The present invention relates to peptides representative of polypeptides of interest and antibodies directed thereagainst. More specifically, the present invention relates to methods, systems and kits for generating and utilizing such peptides and antibodies.

Immunoassays are the most commonly used type of diagnostic assay and still one of the fastest growing technologies used for detection, quantification and characterization of biomolecules. Although, bioassays based phenotypic screening, receptor binding and enzymatic activity are also commonly practiced, such bioassays cannot, in many instances, offer the same unlimited applicability and specificity of immunoassays.

Immunoassay parameters, including, antibody specificity, cross-reactivity and labeling are continuously researched in efforts to improve the resolving power of immunoassays [Marks et al. (1992) Biotechnology 10:779-783, Soderlind et al. (1999) Immunotechnology 4:279-285, Ohlin et al. (1996) Mol. Immunol. 33:47-56 and Hemminiki et al. (1998) Immunotechnology 4:59-69].

### Antibody engineering

***Chain shuffling:*** The principle of shuffling gene segments encoding individual or combinations of complementarity-determining regions (CDRs) or entire variable (V) regions of an antibody is to create variation, which can be used to improve antibody affinity [Marks et al. (1992) Biotechnology 10:779-783, Soderlind et al. (1999) Immunotechnology 4:279-285 and Soderlind et al. (2000) 18:852-6]. One of the first examples was the gene shuffling of a human phage antibody specific for the hapten 2-phenyloxalozone. This was achieved by sequentially replacing the heavy and light chain V-region genes with repertoires of V-region genes obtained from non-immunized donors, resulting in a 300-fold increase in affinity.

***Phage display:*** Engineered phage particles, which express a fusion protein consisting of an antibody fragment and a coat-protein can be used for antibody selection. Such phage particles are affinity-purified on immobilized antigen and following amplification in a bacterial host, used for selecting antibodies from large repertoires (e.g., libraries) [Barbas et al. (1991) Proc. Natl. Acad. Sci. USA 88:7978-7982]. Two types of repertoires are used; "immunized repertoires" and "naive repertoires" [Hoogenboom et al. (1998) Immunotechnology 4:1-20]. The main advantage of using an immunized repertoire is that antibodies selected are expected to have high affinities, while it's major drawback is the need for a new repertoire for each new antigen. In this respect, naive libraries are practical, since once constructed they can then be used almost indefinitely against a diverse range of antigens. However, naive libraries have to be very large in order to yield antibodies with reasonable affinities.

***Artificial antibodies:*** Artificial antibodies are a recent development of non-biological alternatives to antibodies, also referred to as "plastibodies" [Haupt et al. (1998) Trends Biotech. 16:468-475]. The plastibody principle is based on molecular imprinting, namely, a recognition site, which is molded directly in a polymer, to thereby mimic the binding site of a natural antibody. Such polymeric constructs are used in molecularly imprinted sorbent assays (MIA) [Vlatakis et al. (1993) Nature 361:645-647], against for example theophylline and diazepam. Plastibodies exhibit a cross-reactivity profile, towards structurally related drugs, similarly to bona fide antibodies. The major problem in the design of molecular alternatives to antibodies is that only low-affinities variants are obtained, probably due to the rigidity of the plastibody recognition site.

Other examples of molecular constructs with 'antibody-like' properties are the 'knottin scaffolds' or the Z domain of the α-helical bacterial receptor. The Z domain does not depend on intramolecular disulfide bridges, and is highly stable at extreme pH and heat conditions, thus making knottin scaffolds valuable in applications such as diagnostics and affinity purification. Although promising, the Z approach suffers from high dissociation constants and as such it is currently limited in applications.

### Immunogens

***Whole protein immunogens:*** A whole protein injection is the preferred method of obtaining high affinity antibodies capable of recognizing the antigen native form. Antibodies which are capable of recognizing the native antigen are frequently used in diagnostics, vaccination and the emerging field of antibody-based therapy. However, various difficulties are associated with whole protein immunogens such as extraction of sufficient amounts of accurately folded protein. Furthermore the use of large protein molecules as immunogens produces antisera containing polyclonal antibodies to the numerous epitopes of the large protein molecules, although monoclonal antibody techniques utilizing whole proteins or large portions thereof as immunogens have been useful in narrowing the immunological response to such immunogens. However, a standalone monoclonal antibody technology is extremely time consuming and yields only a relatively small number of antibodies, which are capable of recognizing the immunogens specifically. Moreover, even when successful, such techniques cannot predict the biochemical identity of the antigenic epitope.

***Synthetic peptide immunogens:*** Synthetic peptide vaccines have been actively researched over the past two decades (Arnon, 1991; Steward and Howard, 1987). However, results show that only a very small portion of monoclonal antibodies raised against short to moderate length peptides derived from the native protein recognize the synthetic peptide immunogens as well as the intact native protein [Arnheiter et al. (1981) Nature 294:278-280]. Furthermore, dissociation constants displayed by the immunocomplexes is oftentimes rather high.

Conjugating synthetic peptides to carriers has been attempted in efforts of improving the affinity of antibodies raised against such synthetic peptides to the native protein [Mariani M., et al. (1987) Mol. Immunol. 24:297-303]. For example Marianni and co-workers conjugated a synthetic peptide corresponding to amino acid residues 166-174 of human chorionic somatomammotropin (hCS) amino acid sequence to elicit monoclonal antibody response to the native hCS molecule. Selected antibody clones were characterized for isotype and affinity. As expected antibodies produced against carrier conjugated hCS peptides showed, on an average, a 1000-folds higher affinity towards the native hCS protein as compared to antibodies generated against non-conjugated peptides. Although promising, this approach oftentimes generates antibodies which are incapable of binding an antigen present in, or derived from, a biological sample.

Several additional approaches for developing synthetic peptide immunogens are known in the art. For example, U.S. Pat. No: 6,261,569 teaches of partially or completely retro-inverso modified antigen analogues, which are capable of mimicking the immunological activity of a native peptide antigen. Although as disclosed in U.S. Pat. No: 6,261,569, these analogues induced the production of antibodies, which recognize a native peptide antigen when administered as an immunogen to an immunocompetent host, retero inversion of peptide antigens has met with limited success in the field of vaccination. Limitations in knowledge concerning antigen-antibody binding prevent accurate predictions as to the nature and binding efficiencies of antibodies elicited against an inverso, retro or retro-inverso peptide. 'The notion of retro inverso antigen analogues was further challenged by Lerner and co-workers (Lerner, 1984) who reported that antibodies generated against native, retro-, inverso- and retro-inverso forms of an influenza virus haemagglutinin peptide were not cross-reactive with the native peptide antigen.

There is thus a widely recognized need for, and it would be highly advantageous to have, peptides representative of a protein of interest, and high affinity antibodies directed thereagainst and methods of generating and using same for detecting, quantifying and/or characterizing polypeptides, such as for example, proteins contained in a biological sample.

### SUMMARY OF THE INVENTION

According to one aspect of the present invention there is provided a method of generating a set of amino acid sequences representative of at least one polypeptide of interest, the method comprising: (a) computationally generating a plurality of proteolytic cleavage products from the at least one polypeptide of interest; (b) computationally analyzing the plurality of proteolytic cleavage products according to at least one parameter defining a characteristic of an amino acid sequence; and (c) selecting a set of proteolytic cleavage products from the plurality of proteolytic cleavage products according to predetermined criteria for each of the at least at least parameter, thereby generating the set of amino acid sequences representative of the at least one polypeptide of interest.

According to another aspect of the present invention there is provided a computer readable storage media comprising a database of amino acid sequences corresponding to at least one polypeptide of interest, the database of amino acid sequences being generated by: (a) computationally generating a plurality of proteolytic cleavage products from the at least one polypeptide of interest; (b) computationally analyzing the plurality of proteolytic cleavage products according to at least one parameter defining a characteristic of an amino acid sequence; and (c) storing a sequence of each of the proteolytic cleavage products thereby generating the database of amino acid sequences.

According to yet another aspect of the present invention there is provided a system for generating a database of amino acid sequences of at least one polypeptide of interest, the system comprising a processing unit, the processing unit executing a software application configured for: (a) generating a plurality of protcolytic cleavage products from the at least one polypeptide of interest; and (b) analyzing the plurality of proteolytic cleavage products according to at least one parameter defining a characteristic of an amino acid sequence.

According to still another aspect of the present invention there is provided a kit for quantifying at least one polypeptide of interest, the kit comprising a plurality of peptides being generated according to information derived from computational analysis of the at least one polypeptide of interest, the computational analysis including generating a plurality of proteolytic cleavage products from the at least one polypeptide of interest.

According to further features in preferred embodiments of the invention described below, the plurality of peptides are labeled.

According to still further features in the described preferred embodiments the plurality of peptides are attached to a solid substrate.

According to still further features in the described preferred embodiments the plurality of peptides is contained in an individual container.

According to still further features in the described preferred embodiments the peptides are mixed in a single container.

According to still further features in the described preferred embodiments the plurality of peptides are generated via peptide synthesis or proteolytic cleavage of the at least one polypeptide of interest.

According to an additional aspect of the present invention there is provided a kit for quantifying at least one polypeptide of interest, the kit comprising a plurality of antibodies each capable of specifically recognizing at least one peptide of a plurality of peptides, the plurality of peptides being generated according to information derived from computational analysis of the at least one polypeptide of interest, the computational analysis including generating a plurality of proteolytic cleavage products from the at least one polypeptide of interest.

According to still further features in the described preferred embodiments the plurality of antibodies are labeled.

According to still further features in the described preferred embodiments the plurality of antibodies are attached to a solid substrate.

According to still further features in the described preferred embodiments the plurality of antibodies is contained in an individual container.

According to still further features in the described preferred embodiments the plurality of antibodies are mixed in a single container.

According to yet additional aspect of the present invention there is provided a method of quantifying at least one polypeptide of interest in a biological sample, the method comprising: (a) contacting the biological sample with at least one proteolytic agent, so as to obtain a proteolysed biological sample; (b) contacting the proteolysed biological sample with at least one antibody and at least one peptide of a plurality of peptides, wherein the antibody is capable of specifically binding the at least one peptide of the plurality of peptides, and further wherein the plurality of peptides are generated according to information derived from computational analysis of the at least one polypeptide of interest, the computational analysis including generating a plurality of proteolytic cleavage products from the at least one polypeptide of interest; and (c) detecting presence, absence and/or level of antibody binding to thereby quantify the at least one polypeptide of interest in the biological sample.

According to still further features in the described preferred embodiments the at least one antibody is attached to a solid substrate.

According to still further features in the described preferred embodiments the solid substrate is configured as a microarray and the at least one antibody includes a plurality of antibodies each attached to the microarray in a regio-specific manner.

According to still further features in the described preferred embodiments the at least one antibody and/or the at least one peptide is labeled and whereas step (c) is effected by quantifying the label.According to still further features in the described preferred embodiments the at least one peptide is attached to a solid substrate.

According to still further features in the described preferred embodiments the solid substrate is configured as a microarray and each of the plurality of peptides is attached to the microarray in a regio-specific manner.

According to still additional aspect of the present invention there is provided a method of generating at least one antibody specific to a polypeptide of interest, the method comprising using at least one peptide to generate the at least one antibody specific to the polypeptide of interest, wherein the at least one peptide is generated according to information derived from computational analysis of the polypeptide of interest, the computational analysis including generating a plurality of proteolytic cleavage products from the polypeptide of interest.

According to still further features in the described preferred embodiments, computational analysis further includes analysis of the plurality of proteolytic cleavage products according to at least one parameter defining a characteristic of an amino acid sequence and selection of a set of proteolytic cleavage products from the plurality of proteolytic cleavage products according to predetermined criteria for each of the at least at least parameter.

According to still further features in the described preferred embodiments the plurality of proteolytic cleavage products are generated according to a proteolytic cleavage pattern of at least one proteolytic agent.

According to still further features in the described preferred embodiments the at least one proteolytic agent is selected from the group consisting of a proteolytic enzyme and a proteolytic chemical.

According to still further features in the described preferred embodiments the proteolytic enzyme is selected from the group consisting of trypsin, chymotrypsin, subtilisin, pepsin, V8 protease, thrombin and elastase.

According to still further features in the described preferred embodiments the proteolytic chemical is selected from the group consisting of cyanogen bromide and 2-nitro-5-thiocyanobenzoate.

According to still further features in the described preferred embodiments the at least one parameter defining the characteristic of the amino acid sequence is selected from the group consisting of molecular weight, amino acid composition, hydrophobicity, hydrophilicity, charge, secondary structure, heterogeneity, length, post-translational modifications, polarity, solubility, amphipathic nature, sequence and immunogenicity.

The present invention successfully addresses the shortcomings of the presently known configurations by providing a novel approach for producing immunogens and antibodies directed thereto and novel immunoassays utilizing same.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 illustrates a system designed and configured for generating a database of amino acid sequences representative of a protein of interest according to the teachings of the present invention.
FIG. 2 illustrates a remote configuration of the system described in Figure 1.
FIG. 3 illustrates a calibration curve of the enzyme-linked Elisa assay of the present invention as obtained by adding serial dilutions of peptide probe to a fixed quantity of antibodies recognizing the peptide probe. Data is fitted by hyperbolic descending. The horizontal lines represent the optical density of triplicate digested samples of P-glycoprotein expressing CHO cells (lower line) and wild type CHO cells (upper line).
FIG. 4 illustrates the use of an antibody matrix designed and constructed according to the teachings of the present invention.

### DETAILED DESCRIPTION OF THE PREFERRED EMBODIMENTS

The present invention is of peptides representative of polypeptides of interest and antibodies directed thereagainst. Specifically, the peptides generated according to the teachings of the present invention can be used to generate antibodies useful for detecting quantifying and characterizing proteins contained in, or derived from a biological sample.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings described in the Examples section. The invention is capable of other embodiments or of being practiced or carried out in various ways. Also, it is to be understood that the phraseology and terminology employed herein is for the purpose of description and should not be regarded as limiting.

Antibodies are widely used in the fields of diagnostics, vaccination and therapy.

To date, most approaches for generating antibodies use purified intact proteins or synthetic peptide antigens as preferred immunogens. While the former is limited by the need to extract sufficient amounts of precisely folded protein, an essentially laborious and expensive method, the latter suffers from low immunogenicity and high dissociation constants.

The present invention provides a novel approach for producing peptides representative of polypeptides of interest and antibodies directed thereagainst, which antibodies are capable of specifically recognizing an antigen while being of minimal cross-reactivity potential.

As described hereinunder and in the Examples section which follows, the present invention provides novel peptide antigens which when administered to an immunocompetent host are capable of effectively inducing the production of protein-specific antibodies which can be used to detect the protein in samples, which contain low protein concentrations and/or low exposure of protein-specific antigenic regions.

The phrase "amino acid sequences" refers herein to an oligopeptide, peptide, polypeptide, or protein sequence and fragments thereof. Such molecules can be naturally occurring or synthetic.

The phrase "polypeptide-of-interest" is used herein in reference of any naturally occurring polypeptide or antigenic fragment thereof. The polypeptide-of-interest in accordance with the present invention includes, but is not limited to pathogen derived polypeptides such as, poliomyelitis, hepatitis B, foot and mouth disease of livestock, tetanus, pertussis, HIV, cholera, malaria, influenza, rabies or diphtheria causing agents, or toxins such as robustoxin, heat labile toxin of pathogenic Escherichia coli strains, Shiga toxin from Shigella dysenteriae, polypeptides derived from multi-drug resistant strains of staphyloccocus Aureus and the like; degenerative diseases associated antigens such as the Amyloid-β-protein of Alzheimer's disease; and pregnancy and fertility associated antigens, which antigens include for example human chorionic gonadotropin and gonadotropin releasing hormone.

The polypeptide of interest can also be a tumor associated antigen such as growth factors, growth factor receptors, as well as oncogene-encoded proteins, which are expressed at significantly increased or decreased levels on tumor cells, examples include but are not limited to transferrin growth factor (Tf), Epidermal growth factor receptor (HER 1-3), Fibroblast growth factor receptor (FGFR-1), Epidermal growth factor (EGF), p21-Ras, p53, ERa, bcl-2 and the like or oncofetal antigens such as α-fetoprotein (AFP) and carcinoembryonic antigen (CEA), MAGE-1, MAGE-3, BAGE, GAGE-1,2 and the like. Alternatively the polypeptide of interest can be a tumor specific antigen, which antigens are unique to tumor cells, and do not occur on normal cells in the body. Alternatively, the polypeptide of interest can be one associated with DNA repair, or with the suppression or enhancement of apoptosis or cell senescence.

Thus, according to one aspect of the present invention there is provided a method of generating a set of amino acid sequences representative of one or more polypeptides of interest.

The method according to this aspect of the present invention is effected by several steps.

First, a plurality of proteolytic cleavage products of the at least one polypeptide of interest are computationally generated according to a known proteolytic cleavage pattern of one or more proteolytic agent.

Following computer generation, the proteolytically cleaved amino acid sequences derived from the polypeptide of interest are computationally analyzed according to one or more parameters, which define amino acid sequence characteristics.

These parameters are used individually or in combination according to predetermined criteria to select a set of amino acid sequences derived from the at least one polypeptide of interest.

Sequences that represent the one or more polypeptides of interest can be stored in a database which can be generated by a suitable computing platform. Amino acid sequences of the present invention can be further used to generate peptides and antibodies directed thereagainst, which can be packed in diagnostic kits and implemented in various therapeutic and diagnostic methods.

The proteolytic cleavage products described above can be computationally generated according to a known'proteolytic cleavage pattern of one or more proteolytic agents. This can be effected using any processing software capable of recognizing proteolytic cleavage sites within an amino acid sequence and generating the amino acid sequences of such proteolysis.

It will be appreciated that although the use of a dedicated software application, such as Sciprot (available from www.asiaonline.net.hk/∼twcbio/DOCS/1/scPrtein.htm), the GCG package (Genetics Computer Group, Wisconsin) or Macvector (available from www.accelrys.com/products/macvector/) is preferred, computational proteolysis of amino acid sequences can also be generated using non-dedicated software applications such as for example, a text edit application (e.g., Word available from Microsoft Inc.). Such a non-dedicated application can be used to recognize specific alphanumeric character sequences (i.e., cleavage sites) and to generate sequences of amino acids, which correspond to the cleavage products according to commands provided by the user (e.g., a Word Macro).

The proteolytic agent can be any agent, which is capable of cleaving polypeptides between specific amino acid residues (i.e., the proteolytic cleavage pattern).

According to one embodiment of this aspect of the present invention a proteolytic agent is a proteolytic enzyme. Examples of proteolytic enzymes, include but are not limited to trypsin, chymotrypsin, V8 protease, pepsin, subtilisin, thrombin, elastase, caspase-1, caspase-2, caspase-3, caspase-4, caspase-5, caspase-6, caspase-7, caspase-8, MetAP-2, adenovirus protease, HIV protease and the like.

According to another embodiment of this aspect of the present invention a proteolytic agent is a proteolytic chemical such as cyanogen bromide and 2-nitro-5-thiocyanobenzoate.

It will be appreciated that although any proteolytic agent known in the art can be used by the present invention, proteolytic reagents or combinations thereof, which enable complete substrate proteolysis are preferred.

Following computer generation, the proteolytically cleaved amino acid sequences derived from the polypeptide of interest are computationally analyzed according to one or more parameters; which define amino acid sequence characteristics.

Examples of such parameters include but are not limited to molecular weight, amino acid composition, hydrophobicity, hydrophilicity, charge, secondary structure, heterogeneity, length, post-translational modifications, polarity, solubility, amphipathic nature, sequence and immunogenicity.

Each of the peptide products computationally generated is classified according to one or preferably several parameters, which are described in detail below.

Each parameter can be considered separately according to predetermined criteria or in combination with other parameters used, in which case, each parameters is also weighted according to its importance. In any case, each of the peptide products is scored. The score of each peptide may be an absolute score, in which case peptides, which score above a predetermined threshold are selected, or alternatively, the score can be proportional in which case highest scoring peptides are selected.

Although sequence analyses can be effected using various protein analysis softwares, analysis may also be effected at least in part, using other software applications not dedicated for such use. Examples of non-dedicated software applications which can be used include spread-sheet software, such as Microsoft Excel (available from Microsoft incorporation).

The following describes in detail parameters, which can be used by the present invention to qualify peptide sequences.

***Homology level:*** The sequences of the computer generated peptide products are compared with protein databases in order to identify substantially non-homologous, (protein-unique) sequences. Preferably, sequences are compared to all known protein databases, employing a sequence alignment algorithm such as BLAST (Basic Local Alignment Search Tool, available through www.ncbi.nlm.nih.gov/BLAST) or the Smith-Waterman algorithm. Sequences that display low homology to database sequences, e.g., not exceeding 40%, preferably not exceeding 30%, more preferably not exceeding 20%, most preferably not exceeding 10%, or presently preferred displaying 0-5% homology are selected or given a high score depending on the type and number of parameters used for qualification.

***Immunogenicity:*** The computer generated peptide products can also be analyzed for their ability to induce a specific immunogenic response, specifically, an antibody response. Various sequence analysis softwares are known in the art, which provide an immunogenicity index according to, for example, the Jameson-Wolf algorithm. Examples include, but are not limited to, Sciprot (available from www.asiaonline.net.hk/∼twcbio/DOCS/1/scPrtein.htm) and Macvector (available from www.accelrys.com/products/macvector/) as well as the widely utilized GCG package (Genetics Computer Group, Wisconsin).

Immunogenicity is determined, at least in part, by the following properties of the immunogen:

Foreignness- in order to elicit an immune response, amino acid sequences must be recognized as non-self by the immune system of the immunocompetent host. Generally, the greater the phylogenetic distance, between the two species (the species from which the antigen was extracted, and the immunocompetent host species), the greater the genetic and therefore the antigenic disparity between them.

Molecular size- there is a correlation between the size of an amino acid sequence and its immunogenicity. As such, amino acid sequences having a molecular mass of at least1000 daltons (Da), more preferably at least 5000 Da, even more preferably at least 10,000 Da and most preferably have a molecular mass approaching 100,000 Da are favored by the present invention and as such are either selected or given a high score.

Chemical composition and heterogeneity- In general, homopolymers (i.e., polymers composed of a single amino acid) tend to lack immunogenicity, regardless of their size. Copolymers of sufficient size, containing two or more different amino acids, are immunogenic. Furthermore, all four levels of protein organization-primary, secondary, tertiary and quaternary-contribute to the structural complexity of a polypeptide and hence affect its immunogenicity.

Susceptibility to antigen processing and presentation- Basically, polypeptides that cannot be degraded and presented with MHC molecules are poor immunogens. Moreover, large insoluble molecules are more immunogenic than small soluble ones, because they are more readily phagocytosed and processed.

***Post-translational modifications:*** The computer generated peptide products can also be analyzed according to the presence, absence and/or level of post-translational modifications. More than 100 different such modifications of amino acid residues are known, examples include but are not limited to phosphorylation, acetylation, methylation, hydroxylation, carboxylation and glycosylation. Sequence analysis softwares which are capable of determining putative post-translational modification in a given amino acid sequence include the NetPhos server which produces neural network predictions for serine, threonine and tyrosine phosphorylation sites in eukaryotic proteins (available through http://www.cbs.dtu.dk/services/NetPhos/), GPI Modification Site Prediction (available through http://mendel.imp.univie.ac.at/gpi) and the ExPASy proteomics server for total protein analysis (available through www.expasy.ch/tools/)

Generally, preferred peptide products are those lacking any post-translational modification sites, since post-translationally modified amino acid sequences are often difficult to purify, and are frequently poor immunogens.

Notwithstanding from the above, peptide products which include post-translational modification, which indicate a biological activity of the polypeptide-of-interest can also be used by the present invention. A very common example is the phosphorylation of OH group of the amino acid side chain of a serine, a threonine, or a tyrosine group in a polypeptide. Depending on the polypeptide, this modification can increase or decrease its functional activity.

***Size* -** Preferred amino acid sequences include fragments of a molecular weight between 600 - 1800 Da and amino acid sequence length between 5 - 12. The size limitation is mainly due to the requirement for minimizing homologies to other polypeptides and technical difficulties associated with synthesizing, purifying and folding large polypeptides (besides the immunogenic-associated size limitation, discussed hereinabove).

The parameters described above are then used individually or in combination to analyze the computationally generated peptide products and to select a set of peptides most suitable for use with the present invention.

As mentioned hereinabove, selection can be effected on the basis of a single parameter or several parameters considered individually or in combination.

In cases where several parameters are examined, a scoring system e.g., a scoring matrix, is preferably used.

Since in some cases immunogenicity may be more important than both post-translational modifications and sizes, while in others, sequence homology might be the most significant parameter, the use of a scoring matrix in which each parameter is weighted enables one to select the most suitable peptides.

Such a scoring matrix can list the various amino acid sequences (of the peptide products) across the X-axis of the matrix while each parameter can be listed on the Y-axis of the matrix. Parameters include both a predetermined range of values from which a single value is selected from each peptide, and a weight. Each peptide is scored at each parameter according to its value and the weight of the parameter.

Finally, the scores of each parameter of a specific peptide are summed and the results are analyzed.

Peptides which exhibit a total score greater than a particular stringency threshold are grouped as members of a peptide set; the higher the score the more stringent the criteria of grouping.

Alternatively, a set of peptides exhibiting the highest scores can also be selected.

The sequences of these peptides, which represent a polypeptide of interest, can then be used to generate a database which can be stored on a computer readable media such as a magnetic, optico-magnetic or optical disk.

In addition to sequence information, such a database can also include additional data relating to database generation, parameters used for selecting peptide sequences, putative uses of the stored sequences, and various other annotations and references which relate to the stored sequences or polypeptide from which they were generated

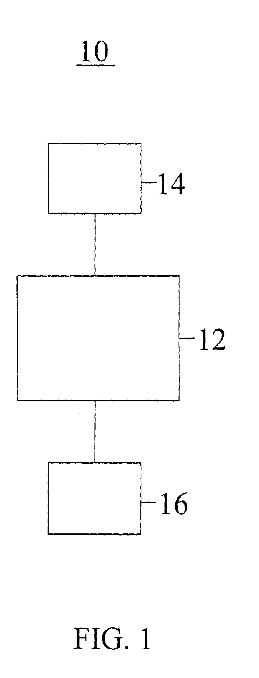

According to another aspect of the present invention and as illustrated in Figure 1, the database of peptide sequences of the present invention is generated by a system designed and configured for such function, which system is referred to hereinunder as system **10.**

System **10** includes a processing unit **12,** which executes a software application designed and configured for generating and preferably analyzing the plurality of proteolytic cleavage products from one or more polypeptides as described hereinabove. System **10** may also include a user input interface **14** (e.g., a keyboard and/or a mouse) for inputting database or database related information, and a user output interface **16** (e.g., a monitor) for providing database information to a user.

System **10** of the present invention may be used by a user to query stored sequences, to retrieve peptide sequences stored therein or to generate peptide sequences from user inputted sequences.

System **10** can be any computing platform known in the art including but not limited to, a personal computer, a work station, a mainframe and the like.

The database generated and stored by system **10** can be accessed by an on-site user of system **10,** or by a remote user communicating with system **10**.

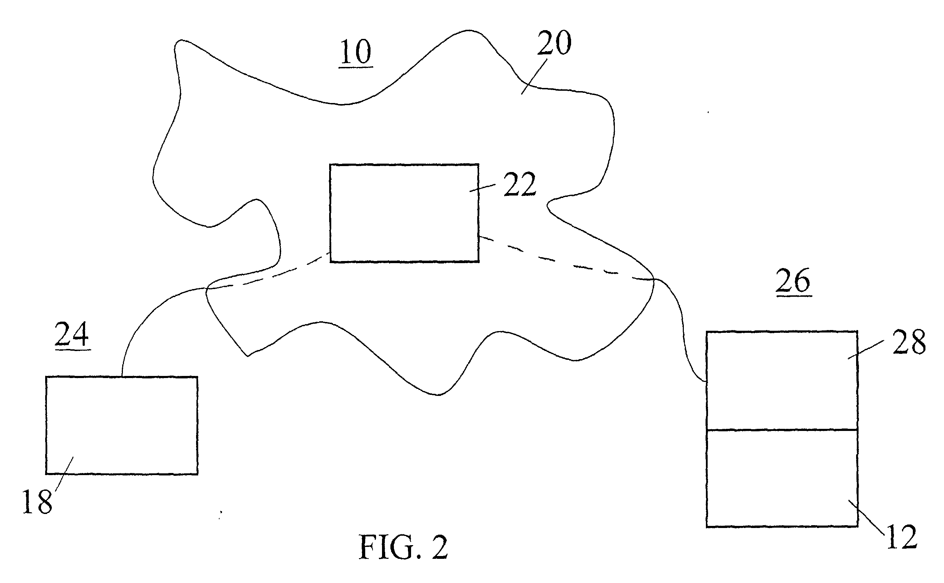

Figure 2 illustrates a remote configurations of system **10** of the present invention.

In such a configuration, the communication between a remote user **18** and processing unit **12** is effected through a communication network **20.** Communication network **20** can be any private or public communication network including, but not limited to, a standard or cellular telephony network, a computer network such as the Internet or intranet, a satellite network or any combination thereof.

As illustrated in Figure 2, communication network **20** includes one or more communication servers **22** (one shown in Figure 2) which serves for communicating data pertaining to the polypeptide of interest between remote user **18** and processing unit **12.**

It will be appreciated that existing computer networks such as the Internet can provide the communication and applications necessary for supporting data communication between any number of sites **24** and remote analysis sites **26.**

For example, using a computer operating a Web browser application and the World Wide Web, any polypeptide of interest can be "uploaded" by user **18** onto a Web site maintained by a database server **28.** Following uploading, database server **28** which serves as processing unit **12** can be instructed by the user to processes the polypeptides as is described hereinabove.

Following such processing, which can be performed in real time, peptide sequence results can be displayed at the web site maintained by database server **28** and/or communicated back to site **24,** via for example, e-mail communication.
Thus, using the Internet, a remote configuration of system **10** can provide protein analysis services to a plurality of sites **24** (one shown in Figure 2). It will be appreciated that this configuration of system **10** of the present invention is especially advantageous in cases where polypeptide analysis can not be effected on-site. For example, laboratories which lack the equipment necessary for executing the analysis or lack the necessary skills to operate it.

The peptide set selected according to the teachings of the present invention can be used to generate peptides either through enzymatic cleavage of the protein from which they were generated and selection of peptides, or preferably through peptide synthesis methods.

Proteolytically cleaved peptides can be separated by chromatographic or electrophoretic procedures and purified and renatured via well known prior art methods.

Synthetic peptides can be prepared by classical methods known in the art, for example, by using standard solid phase techniques. The standard methods include exclusive solid phase synthesis, partial solid phase synthesis methods, fragment condensation, classical solution synthesis, and even by recombinant DNA technology. See, e.g., Merrifield, J. Am. Chem. Soc., 85:2149 (1963), incorporated herein by reference. Solid phase peptide synthesis procedures are well known in the art and further described by John Morrow Stewart and Janis Dillaha Young, Solid Phase Peptide Syntheses (2nd Ed., Pierce Chemical Company, 1984).

Synthetic peptides can be purified by preparative high performance liquid chromatography [Creighton T. (1983) Proteins, structures and molecular principles. WH Freeman and Co. N Y.] and the composition of which can be confirmed via amino acid sequencing.

Due to their protein specificity and immunogenicity, peptides produced according to the teachings of the present invention can be used to generate antibodies characterized by high affinity and specificity.

The peptides generated according to the teachings of the present invention or the antibodies directed thereagainst can be used for both diagnostic and therapeutic purposes.

For example, peptides corresponding to selected amino acid sequences of a protein of interest can be directly administered to an immunocompetent host as immunogens in order to elicit efficient production of antibodies directed at such a protein of interest. Such antibodies would be characterized by high affinity binding and specificity and as such, in cases of disease related protein, such peptides can be used as efficient therapeutic agents.

Alternatively, such peptides can be used to generate antibodies (monoclonal or polyclonal), which in turn can be used for diagnostic purposes.

Various hosts including goats, rabbits, rats, mice, humans and others, may be immunized by peptide injection for the purposes of generating antibodies. Depending on the host species, various adjuvants may be used to increase immunological response. Such adjuvants include, but are not limited to Freund's mineral gels such as aluminum hydroxide, and surface active substances such as lysolecithin, pluronic polyols, polyanions, oil emulsions, keyhole limpet hemocyanin and dinitrophenol.

Monoclonal antibodies may be prepared using any technique which produces antibody molecules by continuous cell lines in culture. These include but are not limited to, the hybridoma technique, the human B-cell hybridoma technique, and the Epstein-Bar-Virus (EBV)-hybridoma technique [Kohler G., et al. (1975) Nature 256:495-497, Kozbor D., et al. (1985) J. Immunol. Methods 81:31-42, Cote R.J. et al. (1983) Proc. Natl. Acad. Sci. 80:2026-2030, Cole S.P. et al. (1984) Mol. Cell. Biol. 62:109-120].

In addition, techniques developed for the production of "chimeric antibodies", the splicing of mouse antibody genes to human antibody genes to obtain a molecule with appropriate antigen specificity and biological activity can be used [Morison S.L. et al. (1984) Proc. Natl. Acad. Sci. 81:6851-6855, Neuberger M.S. et al. (1984) Nature 312:604-608, Takeda S. et al. (1985) Nature 314:452-454]. Alternatively, techniques described for the production of single chain antibodies may be adapted, using methods known in the art.

Antibodies may also be produced by inducing in vivo production in the lymphocyte population or by screening immunoglobulin libraries or panels of highly specific binding reagents as disclosed [Orlandi D.R. et al. (1989) Proc. Natl. Acad. Sci. 86:3833-3837, Winter G. et al. (1991) Nature 349:293-299].

Antibody fragments may also be generated. For example, such fragments include F(ab')2 fragments which may be produced by pepsin digestion of the antibody molecule and the Fab fragments which can be generated by reducing the disulfide bridges of the F(ab')2 fragments. Alternatively, Fab expression libraries may be constructed to allow rapid and easy identification of monoclonal Fab fragments with the desired specificity [Huse W.D. et al. (1989) Science 254:1275-1281].

Various immunoassays may be used for screening antibodies having the desired specificity. Numerous protocols for competitive binding or immunoradiometric assays using either polyclonal or monoclonal antibodies with established specificity are well known in the art. Such immunoassays typically involve the measurement of complex formation between the immunogenic peptide and its specific antibody. Thus the peptides of the present invention may be used for identifying and purifying antibodies.

It will be appreciated that although generation of antibodies against synthetic peptides or cleavage products is preferred, antibodies generated against the whole protein and affinity purified against the synthetic peptides or cleavage products can also be used by the present invention.

Peptides generated according to the teachings of the present invention or antibodies specific thereto can be used to diagnose a variety of diseases including but not limited to diabetes, Parkinson, Alzheimer' disease, HIV, malaria, cholera, influenza, rabies, diphtheria, breast cancer, colon cancer, cervical cancer, melanoma, lung cancer, ovarian cancer, pancreatic cancer, prostate cancer, lymphomas, leukemias and the like and any other diseases which are associated with aberrant expression of multiple antigens.

While most current immuno-diagnostic methods are rate limited by the number of antibodies which can be applied on a given biopsy and sensitivity-limited by masking of protein-specific antigenic regions, the present invention provides an immuno-detection assay which circumvents these limitations.

Thus, according to an additional aspect of the present invention there is provided a method of quantifying at least one polypeptide of interest in a biological sample.

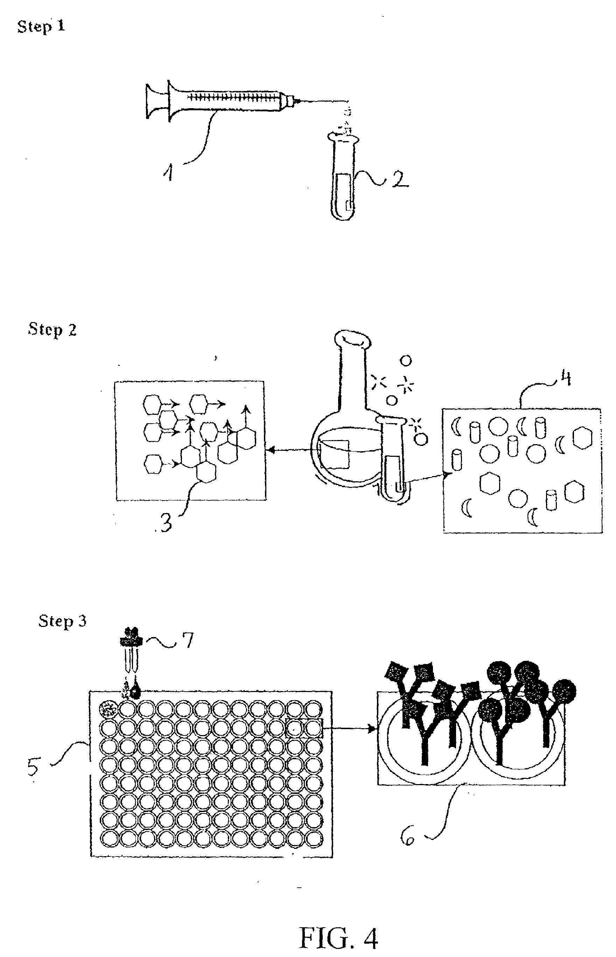

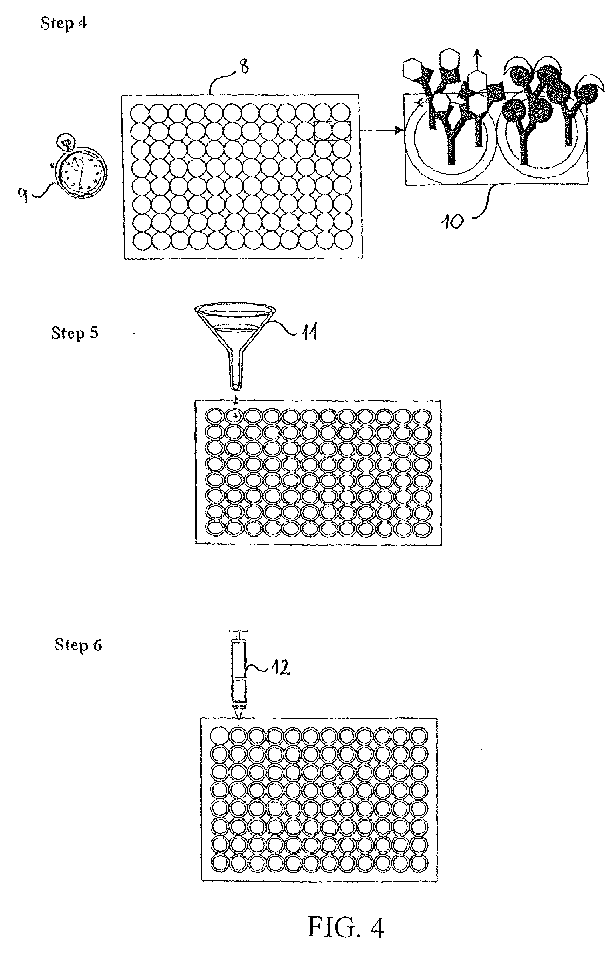

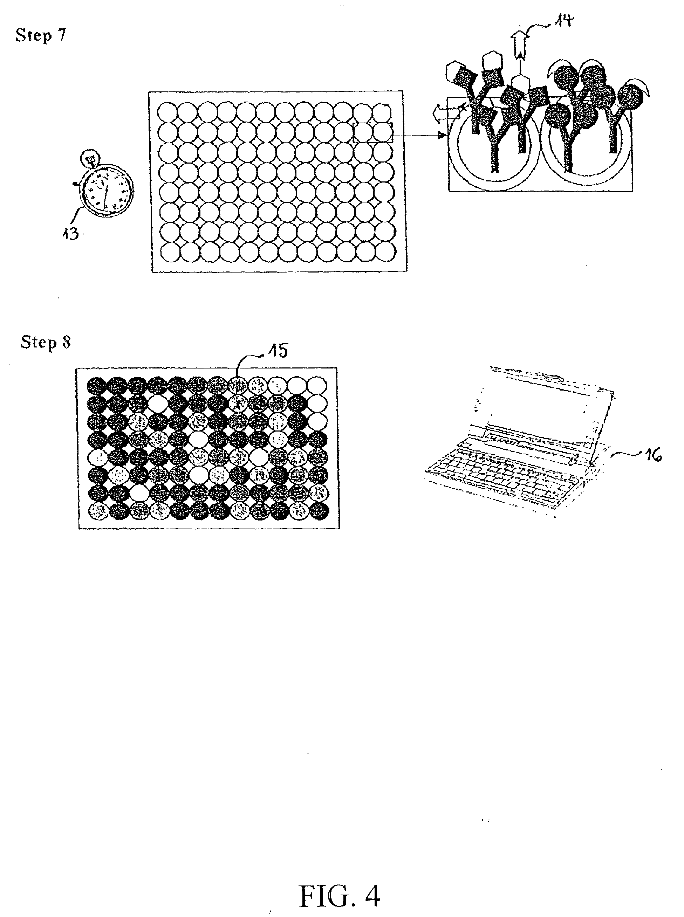

The method includes several method steps, schematically illustrated in Figure 4.

In the first step, a biological sample is obtained. The biological sample as used herein refers to any body sample such as blood (serum or plasma), sputum, ascites fluids, pleural effusions, urine, biopsy specimens, isolated cells and/or cell membrane preparation. Methods of obtaining tissue biopsies and body fluids from mammals are well known in the art.

Retrieved biological samples can be further solubilized using detergent-based or detergent free (i.e., sonication) methods, depending on the biological specimen and the nature of the examined polypeptide (i.e., secreted, membrane anchored or intracellular soluble polypeptide). Oftentimes, protease and phosphatase inhibitors are included within the solubilization buffer, to avoid non-regulated endogenous protease activity, and maintenance of the polypeptides active form.

The solubilized biological sample is contacted with one or more proteolytic agents. Digestion is effected under effective conditions and for a period of time sufficient to ensure complete digestion of the diagnosed polypeptide(s). Agents that are capable of digesting a biological sample under moderate conditions in terms of temperature and buffer stringency are preferred. Measures are taken not to allow non-specific sample digestion, thus the quantity of the digesting agent, reaction mixture conditions (i.e., salinity and acidity), digestion time and temperature are carefully selected. At the end of incubation time proteolytic activity is terminated to avoid non-specific proteolytic activity, which may evolve from elongated digestion period, and to avoid further proteolysis of other peptide-based molecules (i.e., purified peptides and/or antibodies), which are added to the mixture in following steps.

In the next method step the proteolysed biological sample is contacted with one or more antibodies, which are capable of binding one or more proteolytic cleavage products of the examined polypeptide(s) of interest. Such antibodies are capable of specifically binding peptides representative of the polypeptide(s) of interest, which were generated as described hereinabove.

The antibodies are attached to a solid substrate, which may consist of a particulate solid phase such as agarose, sepharose or sephadex beads or a solid substrate configured as an antibody microarray, such as a 96 well plate (see Examples 4 and 5 of the Examples section which follows).

Contacting the proteolysed biological sample with one or more antibodies is effected under conditions suitable for the formation of immune complexes (primary immune complexes).
Immunocomplexes are washed to remove any non-specifically bound antibody species, allowing only those antibodies specifically bound within the primary immune complexes to be detected.

In general monitoring of immunocomplex formation is well known in the art and may be achieved by any one of several approaches. These approaches are generally based on the detection of a label or marker, such as any radioactive, fluorescent, biological or enzymatic tags or labels of standard use in the art. U.S. Patents concerning the use of such labels include U.S. Pat. No. 3,817,837; 3,850,752; 3,939,350; 3,996,345; 4,277,437; 4,275,149 and 4,366,241, each incorporated herein by reference.

The examined polypeptide(s) may be linked to a detectable label, to allow simple detection of this labeled polypeptide, thereby allowing the amount of the primary immune complexes in the composition to be determined. Polypeptide labeling can be effected by labeling the biological sample, prior to, concomitant with or following sample digestion (see Example 3 of the Examples section).

Alternatively, labeled synthetic peptides added to the reaction mixture can be used to quantify binding of the proteolytic products via competitive binding approaches.

The intensity of signal produced in any of the detection methods described hereinabove may be analyzed manually or using a computer program.

In general, polypeptide quantification is preferably effected alongside a calibration curve so as to enable accurate protein determination (see Example 4 of the Examples section below for further detail). Furthermore, quantifying polypeptide(s) originating from a biological sample of a patient is preferably effected by comparison to a normal sample, which sample is characterized by normal expression pattern of the examined polypeptide(s).

It will be appreciated that the detection method described above can also be effected using peptide rather than antibody arrays. In such a case, peptides are attached to a solid support and used along with corresponding antibodies and proteolysed biological samples in competitive binding assays aimed at detecting the presence, absence and/or quantity of specific polypeptides.

It will further be appreciated that in cases of polypeptides which are associated with the formation of anti-self antibodies, such as the case with autoimmune disease associated polypeptides, such peptide arrays can also be used to detect the presence of such autoantibodies, thereby enabling the detection of the disease.

Immunodetection methods of the present invention have evident utility in the diagnosis of various diseases and conditions. In addition, such methods can also be used in non-clinical applications, such as, for example, antigen titering and the like.

The peptides or antibodies generated according to the present invention can be included in a diagnostic or therapeutic kit. For example, peptide sets of specific disease related proteins or antibody populations directed thereagainst can be packaged in a one or more containers with appropriate buffers and preservatives and used for diagnosis or for directing therapeutic treatment. Thus, the peptides or antibodies can be each mixed in a single container or placed in individual containers. Preferably, the containers include a label. Suitable containers include, for example, bottles, vials, syringes, and test tubes. The containers may be formed from a variety of materials such as glass or plastic.

In addition, other additives such as stabilizers, buffers, blockers and the like may also be added.
The peptides or antibodies of such kits can also be attached to a solid support, such as beads, array substrate (e.g., chips) and the like and used for diagnostic purposes. Example 4 and Example 5 of the Examples section describe the use of substrate immobilized antibody arrays designed for such purposes.

Peptides and antibodies included in kits or immobilized to substrates may be conjugated to a detectable label such as an enzyme, in which case the kit also includes substrates and cofactors required by the enzyme to produce a colorimetric reaction (e.g. a substrate precursor which provides the detectable chromophore or fluorophore). Alternatively, the detectable label can be a tag such as an epitope tag , examples of which include but are not limited to a Myc tag, a Flag tag, a His tag, a Leucine tag, an IgG tag, a streptavidin tag and the like, in which case the kit will include an antibody, directed at the epitope and a secondary labeled antibody conjugated to a chromophore or a fluorophore, possibly the epitope directed antibody is labeled.

The kit can also include instructions for determining if the tested subject is suffering from, or is at risk of developing, a condition, disorder, or disease associated with expression of the polypeptide of interest.

The peptides and antibodies directed thereagainst of the present invention are valuable to the fields of biomolecule research, therapy and diagnostics. The ability to simultaneously identify multiple antigens associated with a disease or condition can result in an optimized treatment regimen as well as enable identification of an onset or an early stage of diseases, thereby significantly improving prognosis and treatment.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion. Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### Background

Multi-drug resistance (MDR) represents, a major obstacle in the successful therapy of neoplastic diseases. Studies have demonstrated that this form of drug resistance occurs both in cultured tumor cell lines as well as in human cancers. Recent findings show that numerous molecular mechanisms operate in MDR; from decrease in drug cellular accumulation to the abrogation of drug-induced apoptosis. The most investigated mechanisms for MDR include activation or upregulation of transmembrane proteins, effluxing different chemical substances from the cells (P-glycoprotein is the most characterized efflux pump), activation of the glutathione detoxification system and alterations of genes and proteins involved in the control of apoptosis (especially p53 and Bcl-2).

It has been suggested that expression of MDR associated proteins has a prognostic value to various types of human cancers including leukemias and soft tissue sarcomas. For example, the failure to achieve complete remission in acute myeloid leukemia has been associated with P-glycoprotein expression [Broxterman HJ. et al. (1997) J. int. Med. Suppl. 70:147-51], while P-glycoprotein expression was shown to serve as a molecular marker for response to chemotherapy in bone and soft tissue sarcomas [Stein V et al. (1996) Eur. J. Cancer 32A:86-92].

Further progress towards understanding the clinical importance of MDR associated proteins and P-glycoprotein specifically, is hampered by the lack of validation methods to determine their expression.

### EXAMPLE 1

### Construction of a kit suitable for identifying and quantifying multi-drug resistance associated proteins in a biological sample

The large variety of proteins, which are found to be associated with a condition or a disease, defines a need for a kit which can rapidly evaluate protein antigen in a tissue sample, such as in a biopsy taken from a suspected cancer patient. The kit presented hereinunder is suitable for reacting multiple antigens present in a biological sample with a large number of antibodies at once.

***Step 1 - In-silico extraction of tryptic amino acid sequences:*** Entire protein sequences of P-glycoprotein (GenBank accession number Hs.21330) and Mitoxantrone resistance protein (MXR) (GenBank accession number Hs. 194720), two of the numerous MDR associated proteins, were retrieved from the protein gene bank. The amino acid sequences of P-glycoprotein and MXR were computationally analyzed to obtain tryptic amino acid sequences using the edit/replace function of Microsoft Word (Microsoft Incorporation). Tryptic sequences derived from P-glycoprotein are presented in Table 1 below.

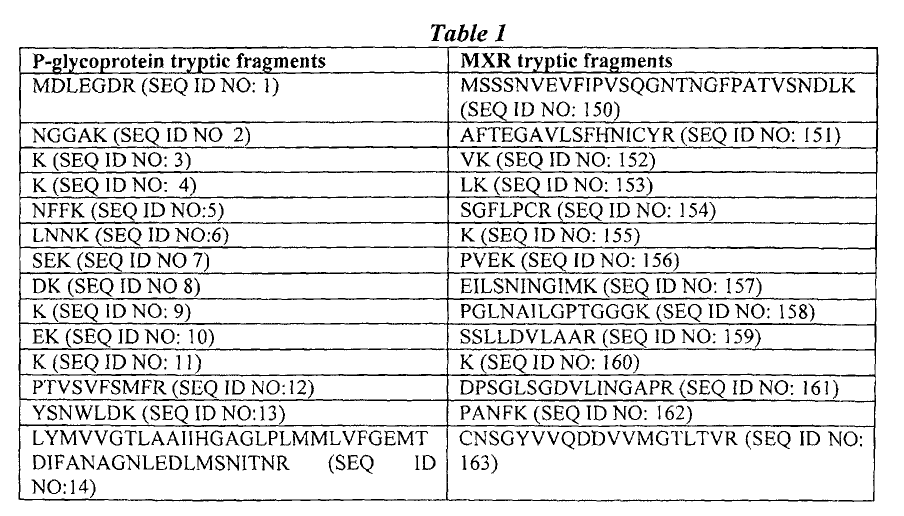

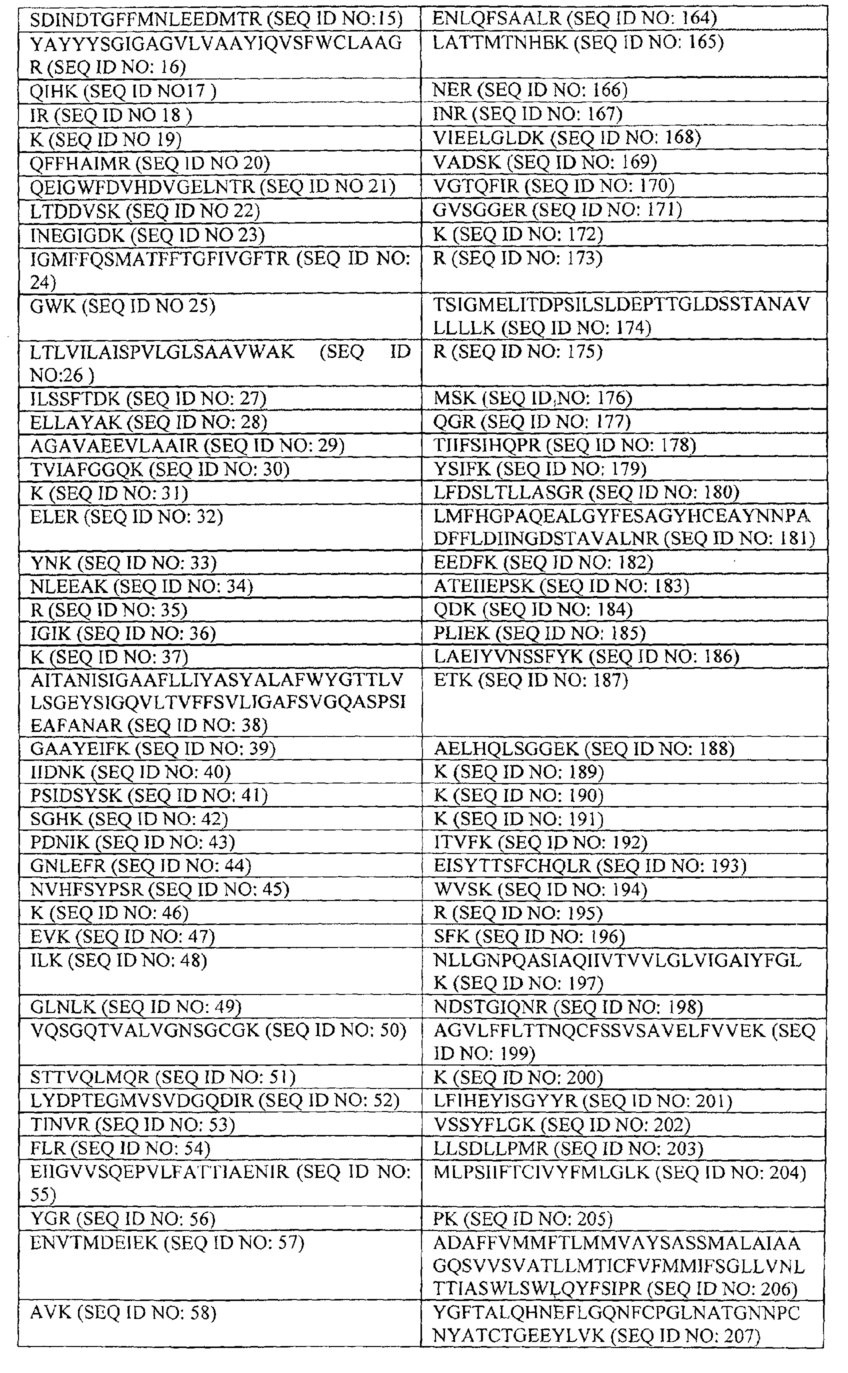

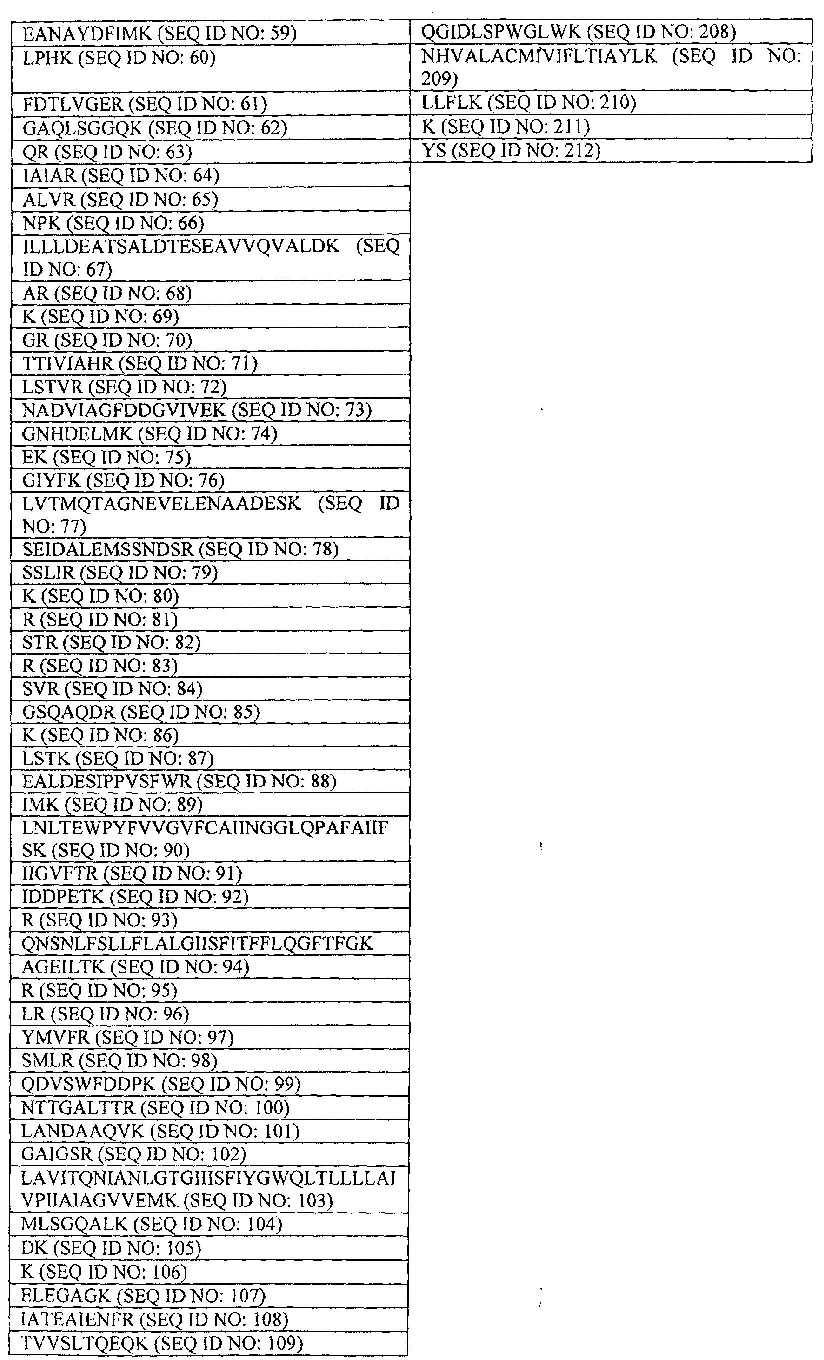

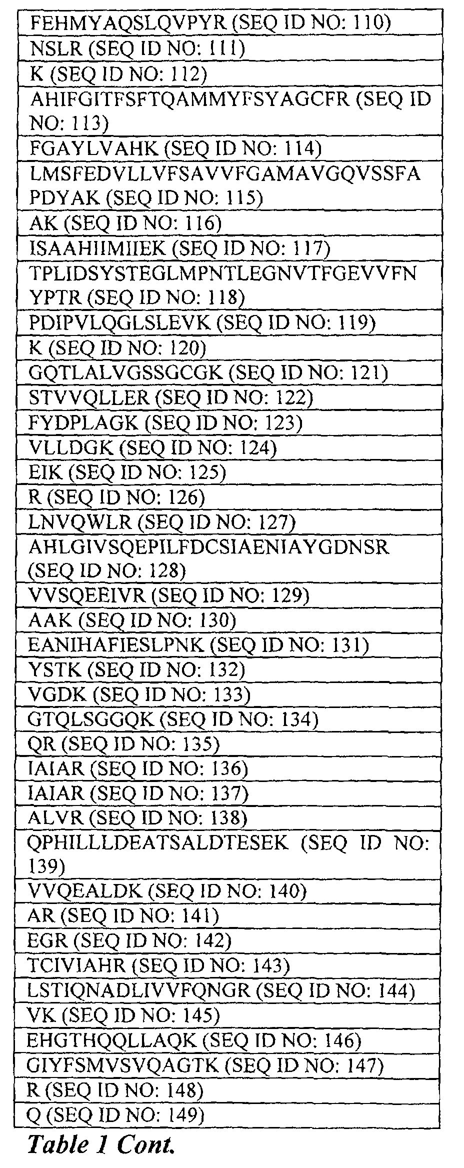

***Step 2 - In silico selection of non-homologous amino acid sequences:*** Computationally extracted tryptic amino acid sequences of P-glycoprotein and MXR protein were scanned for homology to all known protein sequences using the BLAST and Smith-Waterman algorithms by Unix-interfaced GCG server.

Only a portion of the tryptic amino acid sequences listed in Table 1, were found to be unique to each of P-glycoprotein and MXR protein. These sequences, which were not found in any other human protein recorded in the human database (Unigene databank) are listed in Table 2 below.

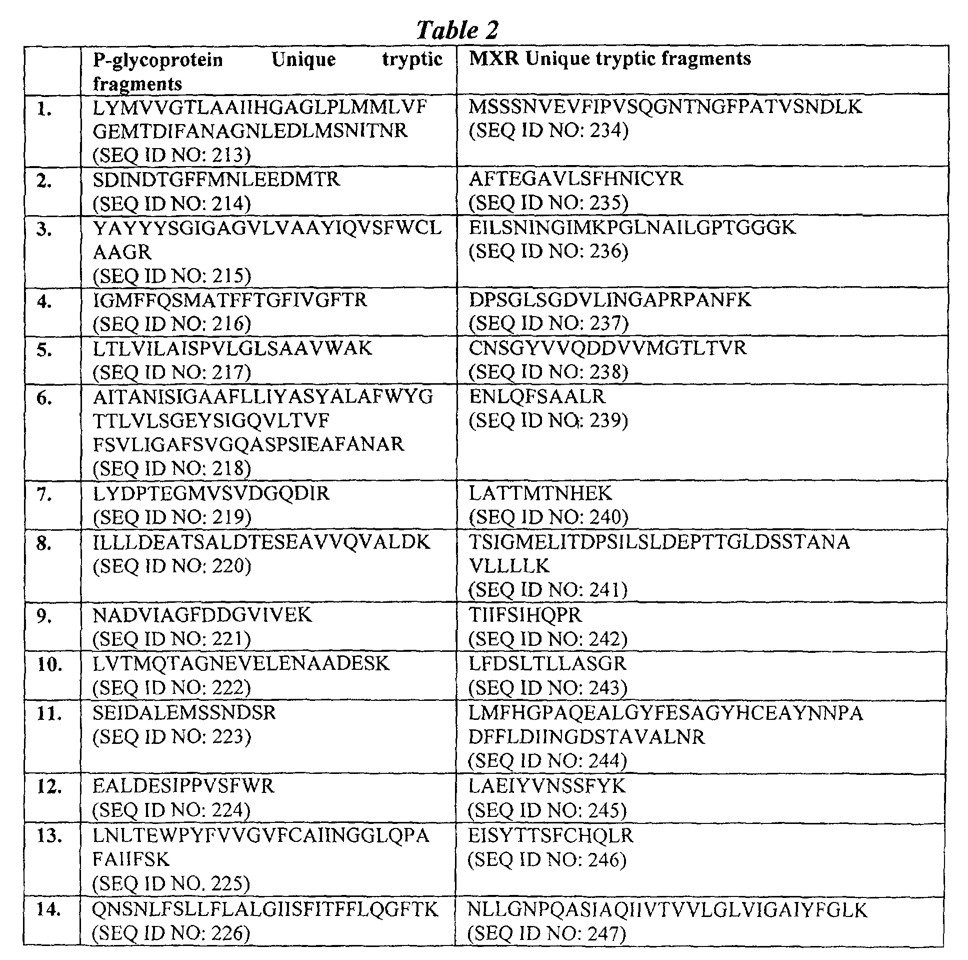

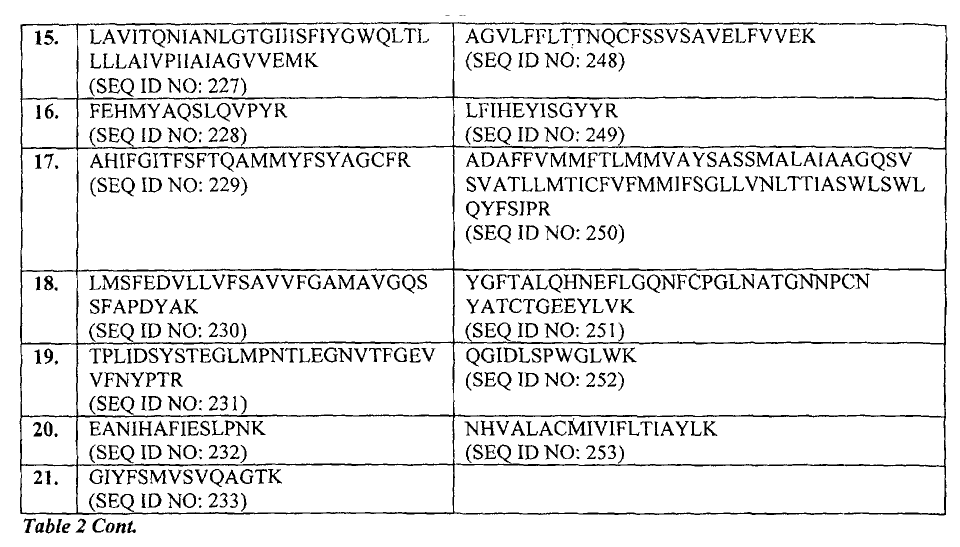

***Step 3 - In silico selection of immunogenic amino acid sequences:*** These unique tryptic amino acid sequences were further analyzed for immunogenicity, by testing parameters such as foreignness, amino acid chemical composition and heterogeneity, peptide molecular weight and susceptibility to antigen processing and presentation.

Following such analysis, several immunogenic sequence candidates were selected from the amino acid sequences presented in Table 2. The candidate sequences selected included peptides: 2, 7, 8, 11 and 19 of the P-glycoprotein list and peptides 5, 8, 10, 13 and 16 of the MXR list.

***Step 4 - Preparation of selected amino acid sequences: Peptide 8 of*** the MXR list and peptides 8 and 19 of the P-glycoprotein list were selected from the above described candidates for further studies. Highly immunogenic portions of these peptides including amino acids 9-21 of MXR peptide 8 and amino acids 12-25 and 13-22 of P-glycoprotein peptides 8 and 19 (respectively) were synthesized and purified by HPLC.

***Step 5 - Generation of antibodies against selected peptides:*** Polyclonal antibodies directed against the selected amino acid sequence of MXR were prepared in rabbit using known techniques, while monoclonal antibodies were obtained against the selected amino acid sequences of the P-glycoprotein.

***Step 6 - Matrix construction and calibration:*** To calibrate the matrix, as to non-specifically bound proteins, polyclonal antibodies against three proteins known to be constitutively expressed in human tissues (i.e., house keeping proteins) were further prepared in rabbits. Each of the control antibodies and the antibodies generated in step 4 above, were conjugated to a solid support, comprised of a multiwell plastic plate (Nunc Immunosorb). Antibodies were left to bind overnight at 4°C at a neutral pH in phosphate-buffered saline (PBS). Subsequent to antibody conjugation, the multiwell plate was washed in PBS containing 1% bovine serum albumine (BSA), and finally stored at 4°C in the presence of 0.01% sodium azide thus generating a matrix of substrate-bound antibodies each specifically directed against an MDR associated peptide.

***Step 7 - preparation of purified labeled tryptic peptides capable of binding the matrix antibodies:*** All tryptic peptides used for generating the antibodies of step 5 were end labeled at the amino terminus by fluorescent labeling, such that a uniform signal is obtained in all the wells or positions in the matrix when these peptides are conjugated with their respective antibodies. This mixture of labeled tryptic polypeptides is capable of competing with MDR associated proteins found in a sample of interest.

### EXAMPLE 2

### A competition assay for quantifying multi-drug resistance associated proteins in a biological sample

This example illustrates a stepwise procedure, which utilizes the matrix described above to identify and quantify multi-drug resistance associated proteins in a biological sample.

A cell culture, which exhibits an MDR phenotype, is dissolved by suspending the sample in a 1-2 % sodium dodecyl sulfate (SDS) containing buffer for one hour. Denatured proteins are precipitated by adding methanol/acetic acid (pH-4) followed by an overnight incubation at -20°C. Thereafter, the protein precipitate is resuspended in 0.05-0.1 % SDS, and trypsin is added to the resuspended sample. Tryptic digestion is allowed to proceed for 16 hours at 37° until complete proteolytic fragmentation of the sample proteins. At the completion of digestion residual tryptic activity is terminated by adding bovine trypsin inhibitor.

Subsequently, a portion of the tryptic-digested sample is added to an antibody matrix, which is prepared as described hereinabove. Incubation is allowed to proceed for 1 hour at room temperature to allow formation of immunocomplexes, following which, the matrix is washed twice with phosphate buffered saline (PBS) containing 1 % BSA, to reduce non-specific binding.

Monitoring specific binding of sample proteins to the matrix is effected using a mixture of fluorescently labeled polypeptides against which the matrix antibodies were raised. In principle, such a peptide mixture is designed to generate a homogeneously fluorescent surface when applied on a fresh matrix, and to provide a reduced signal when applied to a matrix, which has been previously treated with a digested protein sample detectable by the matrix. The intensity of the fluorescent signal obtained from the matrix as correlated to specific positions in the matrix gives a quantitative measure of the amount of protein present in the sample, after considering the amount of protein sample applied to the matrix, and control binding, as determined using signals obtained from three control antibodies described in Example 1.

### EXAMPLE 3

### A direct assay for quantifying multi-drug resistance associated proteins in a biological sample

This assay is aimed at quantifying MDk associated proteins in a biological sample by utilizing fluorescently labeled peptides products of the digested sample. As such, this assay is based on a direct correlation between the level of the fluorescent signal obtained and the level of the protein to be quantified.

A labeled digested sample is applied onto a fresh antibody matrix, and specific immunocomplexes are allowed to form as described in Example 2 of the Examples section. The intensity of fluorescent signal correlated with specific positions in the matrix, gives a quantitative measure of the amount of MDR associated proteins present in the sample, after considering the amount of protein sample applied to the matrix, and control binding, as determined using signals obtained from three control antibodies described in Example 1.

### EXAMPLE 4

### Quantifying P-glycoprotein expression level in membrane fractions of chinese hamster ovarian cell-lines

The following Example illustrates use of an MDR1-specifc matrix, for determining the expression level of P-glycoprotein (i.e., MDR1 gene product) in two different Chinese hamster ovarian cell-lines.

***Method:*** Total membrane protein (1700 µg) obtained from P-glycoprotein expressing CHO cells (Pgp-CHO) and control wild type cells (WT-CHO) was suspended in 1.12 % SDS, precipitated with methanol/acetic acid, incubated at -20°C for 16 hours, resuspended in 0.07 % SDS and digested overnight with N-tosyl-L-phenylalanine chloromethylketone (TPCK)-treated trypsin. Digested protein samples were centrifuged at 14,000 rpm for 25 minutes, and then spin-filtered through a Vivaspin column (Vivascience Ltd, UK), to remove undigested sample.

The digest was then applied onto each well of an MDR1 specific matrix which was precoated with a monoclonal antibody generated against the peptide probe, MPNTLEGNVTK, where all but the C-terminal lysine corresponded to the central section of an informatically derived tryptic digest product of P-glycoprotein (amino acid coordinates 13-22 of SEQ ID NO: 231). Three control wells of the 96 well matrix were pre-coated with 250 µg of C494, a commercially available monoclonal antibody recognizing P-glycoprotein (Dako corporation, USA).

Serial dilutions of the peptide probe were added in duplicates along with the digested samples to each well starting at minimal dilution containing 500 ng of probe peptide. The matrix was further supplemented with 10 ng of biotinylated probe peptide, and the resulting mixture was incubated for 2 hr room temperature.

Following incubation, the matrix was washed with a washing buffer containing 1 % BSA, and subsequently reacted with streptavidin conjugated to horse-radish peroxidase. The obtained color reaction of the peroxidase enzyme with substrate TMB 3,3',5,5' - Tetramethylbenzidine measured the amount of biotin present in each well. Optical density was determined using an Elisa reader.

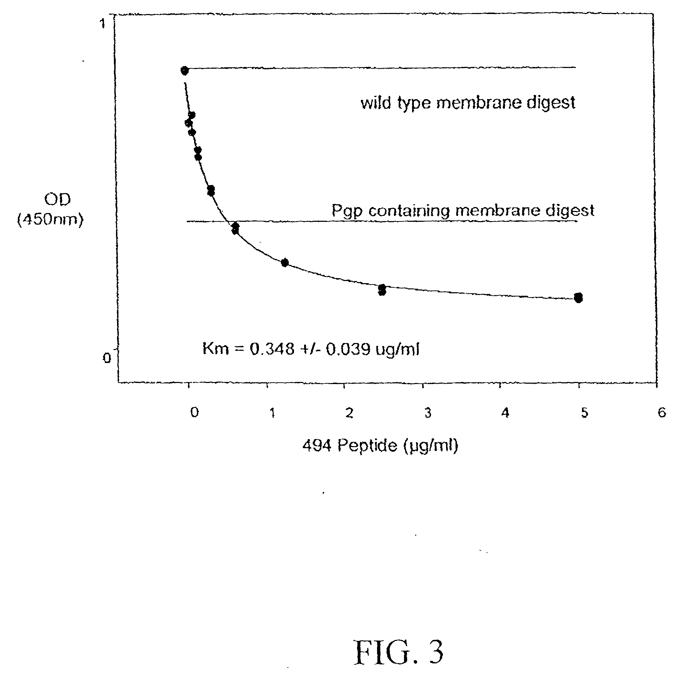

***Results:*** As shown in Figure 3, digested membrane sample of wild-type CHO cells exhibited no reduction in optical density when compared to a control sample, which lacked the labeled digest products, suggesting that wild-type CHO cells are devoid of P-glycoprotein expression. In contrast, digested membrane samples of P-glycoprotein containing cells (Pgp-CHO), exhibited a significant decrease in optical density. As was extrapolated from the calibration curve, 500 µg of original membrane protein represents 0.025 µg of peptide. Given that the molecular weight of the probe peptide is 1000 Da, and that P-glycoprotein has a total molecular weight of 14,460 Da, the content of P-glycoprotein in each well corresponded to 9.33 µg which represents 1.64 % of the cell membrane protein mass. Turnover analysis and enzymatic activity of P-glycoprotein in CHO membranes suggests that the P-glycoprotein membrane content was 5.6 %, implying that the quantitative analysis recovered 29 % of the enzyme originally present.

Control experiments in which a probe peptide was added to the membrane sample prior to the digestive step and subsequently applied on the MDR1-matrix showed an expected recovery of 45 %. The similarity of these two values further validates the accuracy and efficiency of the matrix constructed according to the teachings of the present invention in identifying and quantifying proteins in a biological sample.

### EXAMPLE 5

### A stepwise usage of an antibody matrix to identify and quantify levels of a disease associated protein

Figure 4 illustrate a method of identifying and quantifying disease associated proteins using a protein-specific antibody matrix kit designed and constructed according to the teachings of the present invention.

***Step 1:*** a biopsy (1), which is taken from a patient is solubilized and incubated with a proteolytic agent until complete protein cleavage is achieved (2).

***Step 2:*** The digested sample is mixed with a sample of tagged peptides (3) (represented in the figure by arrow-attached hexagons). The labeled peptides are specific for one or more proteins of interest. These peptides have been selected by scanning a protein database of human sequences for peptides that are unique to protein(s) of interest. Circles, cylinders, non-arrowed hexagons and other shapes (4) represent the peptides in the biopsy digest.

***Step 3:*** The mixture is layered (7) onto a matrix (5), which includes a set of antibodies prepared against the selected peptides. The matrix can be a multiwell plate (e.g., 96 wells) onto which the antibodies are directly or indirectly attached in a regiospecific manner. The inset illustrates the antibodies attached to the wells (6); two wells are depicted, each having a particular antibody attached to it. In addition, the matrix may also include wells of control antibodies, which are prepared against proteins that are ubiquitously present in tissue samples. Furthermore, additional wells may also include labeled peptides at known amounts to serve as standards from which a calibration curve can be derived.

***Step 4:*** The mixture of selected peptides, tagged peptides and attached antibodies(8) is left to form immunocomplexes (9). Inset (10) depicts formed immunocomplexes.

***Step 5:*** The matrix is washed several times with a blocking buffer (11) in order to free non-specifically attached peptides.

***Step 6:*** An enzyme-linked agent specific for the tagged peptides is added (12) and the wells are washed again in order to remove non-specifically bound agent.

***Step 7:*** The matrix is incubated (13) with a substrate, which generates a color reaction (14) when processed by the enzyme-linked agent described in Step 6.

***Step 8:*** The intensity of the color reaction (15) produced is measured in a conventional 96-well plate reader and the data analyzed by a computer program(16) which determines the amount of peptide present in each of the wells.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents, patent applications and sequences identified by their accession numbers mentioned in this specification are herein incorporated in their entirety by reference into the specification, to the same extent as if each individual publication, patent, patent application or sequence identified by their accession number was specifically and individually indicated to be incorporated herein by reference. In addition, citation or identification of any reference in this application shall not be construed as an admission that such reference is available as prior art to the present invention.

## Claims

1. A method of generating a set of amino acid sequences representative of at least one polypeptide of interest, the method comprising:
(a) computationally generating a plurality of proteolytic cleavage products from the at least one polypeptide of interest;
(b) computationally analyzing said plurality of proteolytic cleavage products according to at least one parameter defining a characteristic of an amino acid sequence; and
(c) selecting a set of proteolytic cleavage products from said plurality of proteolytic cleavage products according to predetermined criteria for each of said at least at least parameter, thereby generating the set of amino acid sequences representative of the at least one polypeptide of interest.

2. The method of claim 1, wherein said plurality of proteolytic cleavage products are generated according to a proteolytic cleavage pattern of at least one proteolytic agent.

3. The method of claim 2, wherein said at least one proteolytic agent is selected from the group consisting of a proteolytic enzyme and a proteolytic chemical.

4. The method of claim 3, wherein said proteolytic enzyme is selected from the group consisting of trypsin, chymotrypsin, subtilisin, pepsin, V8 protease, thrombin and elastase.

5. The method of claim 3, wherein said proteolytic chemical is selected from the group consisting of cyanogen bromide and 2-nitro-5-thiocyanobenzoate.

6. The method of claim 1, wherein said at least one parameter defining said characteristic of said amino acid sequence is selected from the group consisting of molecular weight, amino acid composition, hydrophobicity, hydrophilicity, charge, secondary structure, heterogeneity, length, post-translational modifications, polarity, solubility, amphipathic nature, sequence and immunogenicity.

7. A computer readable storage media comprising a database of amino acid sequences corresponding to at least one polypeptide of interest, said database of amino acid sequences being generated by:
(a) computationally generating a plurality of proteolytic cleavage products from the at least one polypeptide of interest; and
(b) computationally analyzing said plurality of proteolytic cleavage products according to at least one parameter defining a characteristic of an amino acid sequence; and
(c) storing a sequence of each of said proteolytic cleavage products thereby generating said database of amino acid sequences.

8. The computer readable storage media of claim 7, wherein said plurality of proteolytic cleavage products are generated according to a proteolytic cleavage pattern of at least one proteolytic agent.

9. The computer readable storage media of claim 8, wherein said at least one proteolytic agent is selected from the group consisting of a proteolytic enzyme and a proteolytic chemical.

10. The computer readable storage media of claim 9, wherein said proteolytic enzyme is selected from the group consisting of trypsin, chymotrypsin, subtilisin, pepsin, V8 protease, thrombin and elastase.

11. The computer readable storage media of claim 9, wherein said proteolytic chemical is selected from the group consisting of cyanogen bromide and 2-nitro-5-thiocyanobenzoate.

12. The computer readable storage media of claim 7, wherein said at least one parameter defining said characteristic of said amino acid sequence is selected from the group consisting of molecular weight, amino acid composition, hydrophobicity, hydrophilicity, charge, secondary structure, heterogeneity, length, post-translational modifications, polarity, solubility, amphipathic nature, sequence and immunogenicity.

13. A system for generating a database of amino acid sequences of at least one polypeptide of interest, the system comprising a processing unit, said processing unit executing a software application configured for:
(a) generating a plurality of proteolytic cleavage products from the at least one polypeptide of interest; and
(b) analyzing said plurality of proteolytic cleavage products according to at least one parameter defining a characteristic of an amino acid sequence.

14. The system of claim 13, wherein said plurality of proteolytic cleavage products are generated according to a proteolytic cleavage pattern of at least one proteolytic agent.

15. The system of claim 14, wherein said at least one proteolytic agent is selected from the group consisting of a proteolytic enzyme and a proteolytic chemical.

16. The system of claim 15, wherein said proteolytic enzyme is selected from the group consisting of trypsin, chymotrypsin, subtilisin, pepsin, V8 protease, thrombin and elastase.

17. The system of claim 15, wherein said proteolytic chemical is selected from the group consisting of cyanogen bromide and 2-nitro-5-thiocyanobenzoate.

18. The system of claim 13, wherein said at least one parameter defining said characteristic of said amino acid sequence is selected from the group consisting of molecular weight, amino acid composition, hydrophobicity, hydrophilicity, charge, secondary structure, heterogeneity, length, post-translational modifications, polarity, solubility, amphipathic nature, sequence and immunogenicity.

19. A kit for quantifying at least one polypeptide of interest, the kit comprising a plurality of peptides being generated according to information derived from computational analysis of the at least one polypeptide of interest, said computational analysis including generating a plurality of proteolytic cleavage products from the at least one polypeptide of interest.

20. The kit of claim 19, wherein said computational analysis further includes analysis of said plurality of proteolytic cleavage products according to at least one parameter defining a characteristic of an amino acid sequence and selection of a set of proteolytic cleavage products from said plurality of proteolytic cleavage products according to predetermined criteria for each of said at least at least parameter.

21. The kit of claim 19, wherein said plurality of proteolytic cleavage products are generated according to information derived from a proteolytic cleavage pattern of at least one proteolytic agent.

22. The kit of claim 21, wherein said at least one proteolytic agent is selected from the group consisting of a proteolytic enzyme and a proteolytic chemical.

23. The kit of claim 22, wherein said proteolytic enzyme is selected from the group consisting of trypsin, chymotrypsin, subtilisin, pepsin, V8 protease, thrombin and elastase.

24. The kit of claim 22, wherein said proteolytic chemical is selected from the group consisting of cyanogen bromide and 2-nitro-5-thiocyanobenzoate.

25. The kit of claim 20, wherein said at least one parameter defining said characteristic of said amino acid sequence is selected from the group consisting of molecular weight, amino acid composition, hydrophobicity, hydrophilicity, charge, secondary structure, heterogeneity, length, post-translational modifications, polarity, solubility, amphipathic nature, sequence and immunogenicity.

26. The kit of claim 19, wherein said plurality of peptides are labeled.

27. The kit of claim 19, wherein said plurality of peptides are attached to a solid substrate.

28. The kit of claim 19, wherein each of said plurality of peptides is contained in an individual container.

29. The kit of claim 19, wherein said plurality of peptides are mixed in a single container.

30. The kit of claim 19, wherein said plurality of peptides are generated via peptide synthesis or proteolytic cleavage of the at least one polypeptide of interest.

31. A kit for quantifying at least one polypeptide of interest, the kit comprising a plurality of antibodies each capable of specifically recognizing at least one peptide of a plurality of peptides, said plurality of peptides being generated according to information derived from computational analysis of the at least one polypeptide of interest, said computational analysis including generating a plurality of proteolytic cleavage products from the at least one polypeptide of interest.

32. The kit of claim 31, wherein said computational analysis further includes analysis of said plurality of proteolytic cleavage products according to at least one parameter defining a characteristic of an amino acid sequence and selection of a set of proteolytic cleavage products from said plurality of proteolytic cleavage products according to predetermined criteria for each of said at least at least parameter.

33. The kit of claim 31, wherein said plurality of proteolytic cleavage products are generated according to information derived from a proteolytic cleavage pattern of at least one proteolytic agent.

34. The kit of claim 33, wherein said at least one proteolytic agent is selected from the group consisting of a proteolytic enzyme and a proteolytic chemical.

35. The kit of claim 34, wherein said proteolytic enzyme is selected from the group consisting of trypsin, chymotrypsin, subtilisin, pepsin, V8 protease, thrombin and elastase.

36. The kit of claim 34, wherein said proteolytic chemical is selected from the group consisting of cyanogen bromide and 2-nitro-5-thiocyanobenzoate.

37. The kit of claim 32, wherein said at least one parameter defining said characteristic of said amino acid sequence is selected from the group consisting of molecular weight, amino acid composition, hydrophobicity, hydrophilicity, charge, secondary structure, heterogeneity, length, post-translational modifications, polarity, solubility, amphipathic nature, sequence and immunogenicity.

38. The kit of claim 31, wherein said plurality of antibodies are labeled.

39. The kit of claim 31, wherein said plurality of antibodies are attached to a solid substrate.

40. The kit of claim 31, wherein each of said plurality of antibodies is contained in an individual container.

41. The kit of claim 31, wherein said plurality of antibodies are mixed in a single container.

42. The kit of claim 31, wherein said plurality of peptides are generated via peptide synthesis or proteolytic cleavage of the at least one polypeptide of interest.

43. A method of quantifying at least one polypeptide of interest in a biological sample, the method comprising:
(a) contacting the biological sample with at least one proteolytic agent, so as to obtain a proteolysed biological sample;
(b) contacting said proteolysed biological sample with at least one antibody and at least one peptide of a plurality of peptides, wherein said antibody is capable of specifically binding said at least one peptide of said plurality of peptides, and further wherein said plurality of peptides are generated according to information derived from computational analysis of the at least one polypeptide of interest, said computational analysis including generating a plurality of proteolytic cleavage products from the at least one polypeptide of interest; and
(c) detecting presence, absence and/or level of antibody binding to thereby quantify the at least one polypeptide of interest in the biological sample.

44. The method of claim 43, wherein said at least one antibody is attached to a solid substrate.

45. The method of claim 44, wherein said solid substrate is configured as a microarray and said at least one antibody includes a plurality of antibodies each attached to said microarray in a regio-specific manner.

46. The method of claim 43, wherein said at least one antibody and/or said at least one peptide is labeled and whereas step (c) is effected by quantifying said label.

47. The method of claim 43, wherein said plurality of peptides are generated by peptide synthesis or proteolytic cleavage of the at least one polypeptide of interest.

48. The method of claim 43, wherein said computational analysis further includes analysis of said plurality of proteolytic cleavage products according to at least one parameter defining a characteristic of an amino acid sequence and selection of a set of proteolytic cleavage products from said plurality of proteolytic cleavage products according to predetermined criteria for each of said at least at least parameter.

49. The method of claim 43, wherein said plurality of proteolytic cleavage products are generated according to information derived from a proteolytic cleavage pattern of at least one proteolytic agent.

50. The method of claim 49, wherein said at least one proteolytic agent is selected from the group consisting of a proteolytic enzyme and a proteolytic chemical.

51. The method of claim 40, wherein said proteolytic enzyme is selected from the group consisting of trypsin, chymotrypsin, subtilisin, pepsin, V8 protease, thrombin and elastase.

52. The method of claim 50, wherein said proteolytic chemical is selected from the group consisting of cyanogen bromide and 2-nitro-5-thiocyanobenzoate.

53. The method of claim 48, wherein said at least one parameter defining said characteristic of said amino acid sequence is selected from the group consisting of molecular weight, amino acid composition, hydrophobicity, hydrophilicity, charge, secondary structure, heterogeneity, length, post-translational modifications, polarity, solubility, amphipathic nature, sequence and immunogenicity.

54. The method of claim 43, wherein said at least one peptide is attached to a solid substrate.

55. The method of claim 54, wherein said solid substrate is configured as a microarray and each of said plurality of peptides is attached to said microarray in a regio-specific manner.

56. A method of generating at least one antibody specific to a polypeptide of interest, the method comprising using at least one peptide to generate the at least one antibody specific to the polypeptide of interest, wherein said at least one peptide is generated according to information derived from computational analysis of the polypeptide of interest, said computational analysis including generating a plurality of proteolytic cleavage products from the polypeptide of interest.

57. The method of claim 56, wherein said computational analysis further includes analysis of said plurality of proteolytic cleavage products according to at least one parameter defining a characteristic of an amino acid sequence and selection of a set of proteolytic cleavage products from said plurality of proteolytic cleavage products according to predetermined criteria for each of said at least at least parameter.

58. The method of claim 56, wherein said plurality of proteolytic cleavage products are generated according to information derived from a proteolytic cleavage pattern of at least one proteolytic agent.

59. The method of claim 58, wherein said at least one proteolytic agent is selected from the group consisting of a proteolytic enzyme and a proteolytic chemical.

60. The method of claim 59, wherein said proteolytic enzyme is selected from the group consisting of trypsin, chymotrypsin, subtilisin, pepsin, V8 protease, thrombin and elastase.

61. The method of claim 59, wherein said proteolytic chemical is selected from the group consisting of cyanogen bromide and 2-nitro-5-thiocyanobenzoate.

62. The method of claim 57, wherein said at least one parameter defining said characteristic of said amino acid sequence is selected from the group consisting of molecular weight, amino acid composition, hydrophobicity, hydrophilicity, charge, secondary structure, heterogeneity, length, post-translational modifications, polarity, solubility, amphipathic nature, sequence and immunogenicity.

63. The method of claim 56, wherein said at least one peptide is generated by peptide synthesis or proteolytic cleavage of the at least one polypeptide of interest.
